(19) Europäisches Patentamt
European Patent Office
Office européen des brevets

(11) Publication number: **0 313 697 B1**

(12) **EUROPEAN PATENT SPECIFICATION**

(45) Date of publication of patent specification: **04.03.92**

(51) Int. Cl.5: **C07C 317/06**, C07D 233/84, C07D 233/90, C07D 257/04, A61K 31/34, A61K 31/19

(21) Application number: **87309553.3**

(22) Date of filing: **29.10.87**

(54) **Leukotriene antagonists.**

(43) Date of publication of application:
**03.05.89 Bulletin 89/18**

(45) Publication of the grant of the patent:
**04.03.92 Bulletin 92/10**

(84) Designated Contracting States:
**AT BE CH DE ES FR GB GR IT LI LU NL SE**

(56) References cited:
**EP-A- 0 068 739**
**EP-A- 0 201 823**

(73) Proprietor: **SMITHKLINE BEECHAM CORPORATION**
**One Franklin Plaza P.O. Box 7929**
**Philadelphia Pennsylvania 19103(US)**

(72) Inventor: **Gleason, John Gerald**
**8 Heron Hill Drive**
**Downingtown Pennsylvania 19335(US)**
Inventor: **Hall, Ralph Floyd**
**1311 Prospect Hill Road**
**Villenova Pennsylvania 19085(US)**
Inventor: **Thomas, Wen-Fu Ku**
**1413 Southwind Way Drescher**
**Pennsylvania 19025(US)**
Inventor: **Perchonock, Carl David**
**5212 Augusta Street Bethesda**
**Maryland 20816(US)**

(74) Representative: **Giddings, Peter John, Dr. et al**
**SmithKline Beecham, Corporate Patents, Mundells**
**Welwyn Garden City, Hertfordshire AL7 1EY(GB)**

EP 0 313 697 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

"Slow Reacting Substance of Anaphylaxis" (SRS-A) has been shown to be a highly potent bronchoconstricting substance which is released primarily from mast cells and basophils on antigenic challenge. SRS-A has been proposed as a primary mediator in human asthma. SRS-A, in addition to its pronounced effects on lung tissue, also produces permeability changes in skin and may be involved in acute cutaneous allergic reactions. Further, SRS-A has been shown to effect depression of ventricular contraction and potentiation of the cardiovascular effects of histamine.

The discovery of the naturally occurring leukotrienes and their relationship to SRS-A has reinforced interest in SRS-A and other arachidonate metabolites. SRS-A derived from mouse, rat, guinea pig and man have all been characterized as mixtures of leukotriene-$C_4$ ($LTC_4$), leukotriene-$D_4$ ($LTD_4$) and leukotriene-$E_4$ - ($LTE_4$), the structural formulae of which are represented below.

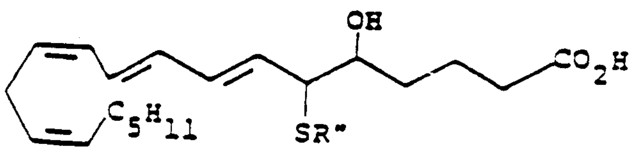

γ Glu
$LTC_4$ R" = Cys-Gly
$LTD_4$ R" = Cys-Gly
$LTE_4$ R" = Cys

Leukotrienes are a group of eicosanoids formed from arachidonic acid metabolism via the lipoxygenase pathway. These lipid derivatives originate from $LTA_4$ and are of two types: (1) those containing a sulfidopeptide side chain ($LTC_4$, $LTD_4$, and $LTE_4$), and (2) those that are nonpeptidic ($LTB_4$). Leukotrienes comprise a group of naturally occurring substances that have the potential to contribute significantly to the pathogenesis of a variety of inflammatory and ischemic disorders. The pathophysiological role of leukotrienes has been the focus of recent intensive studies.

As summarized by Lefer, A.M., Biochemical Pharmacology, 35, 2, 123-127 (1986) both the peptide and non-peptide leukotrienes exert microcirculatory actions, promoting leakage of fluid across the capillary endothelial membrane in most types of vascular beds. $LTB_4$ has potent chemotactic actions and contributes to the recruitment and adherence of mobile scavenger cells to the endothelial membrane. $LTC_4$, $LTD_4$ and $LTE_4$ stimulate a variety of types of muscles. $LTC_4$ and $LTD_4$ are potent bronchoconstrictors and effective stimulators of vascular smooth muscle. This vasoconstrictor effect has been shown to occur in pulmonary, coronary cerebral, renal, and mesenteric vasculatures.

Leukotrienes have been implicated in a number of pulmonary diseases. Leukotrienes are known to be potent bronchoconstrictors in humans. LTC and LTD have been shown to be potent and selective peripheral airway agonists, being more active than histamine. [See Drazen, J.M. et al., Proc. Nat'l. Acad. Sci. USA, 77, 7, 4354-4358 (1980)]. $LTC_4$ and $LTD_4$ have been shown to increase the release of mucus from human airways in vitro. [See Marom, Z. et al., Am. Rev. Respir. Dis., 126, 449-451 (1982).] The leukotriene antagonists of the present invention can be useful in the treatment of allergic or non-allergic bronchial asthma or pulmonary anaphylaxis.

The presence of leukotrienes in the sputum of patients having cystic fibrosis chronic bronchitis, and bronchiectasis at levels likely to have pathophysiological effects has been demonstrated by Zakrzewski et al. [See Zakrzewski, J. T. et al., Prostaglandins, 28, 5, 641 (1984).] Treatment of these diseases constitutes additional possible utility for leukotriene antagonists.

Leukotrienes have been identified in the nasal secretions of allergic subjects who underwent in vivo challenge with specific antigen. The release of the leukotrienes was correlated with typical allergic signs and symptoms. [See Creticos, P.S. et al., New England J. of Med., 310, 25, 1626-1629 (1984).] This suggests that allergic rhinitis is another area of utility for leukotriene antagonists.

The role of leukotrienes and the specificity and selectivity of a particular leukotriene antagonist in an animal model of the adult respiratory distress syndrome was investigated by Snapper et al. [See Snapper, J.R. et al., Abstracts of Int'l Conf. on Prostaglandins and Related Comp., Florence, Italy, p. 495 (June 1986)-.] Elevated concentrations of $LTD_4$ were shown in pulmonary edema fluid of patients with adult respiratory distress syndrome. [See Matthay, M. et al. J. Clin. Immunol., 4, 479-483 (1984).] Markedly elevated leukotriene levels have been shown in the edema fluid of a patient with pulmonary edema after cardiopulmonary bypass. [See Swerdlow, B.N., et al., Anesth. Analg., 65, 306-308, (1986).] LTC and LTD have also been shown to have a direct systemic arterial hypotensive effect and produce vasoconstriction and increased vasopermeability. [See Drazen et al., ibid.] This suggests leukotriene antagonists can also be

useful in the areas of adult respiratory distress syndrome, pulmonary edema, and hypertension.

Leukotrienes have also been directly or indirectly implicated in a variety of non-pulmonary diseases in the ocular, dermatologic, cardiovascular, renal, trauma, inflammatory, carcinogenic and other areas.

Further evidence of leukotrienes as mediators of allergic reactions is provided by the identification of leukotrienes in tear fluids from subjects following a conjunctival provocation test and in skin blister fluids after allergen challenge in allergic skin diseases and conjunctival mucosa. [See Bisgaard, H., et al., Allergy, 40, 417-423 (1985).] Leukotriene immunoreactivity has also been shown to be present in the aqueous humor of human patients with and without uveitis. The concentrations of leukotrienes were sufficiently high that these mediators were expected to contribute in a meaningful way to tissue responses. [See Parker, J.A. et al., Arch Ophthalmol, 104, 722-724 (1986).] It has also been demonstrated that psoriatic skin has elevated levels of leukotrienes. [See Ford-Hutchinson, J. Allergy Clin. Immunol., 74, 437-440 (1984).]. Local effects of intracutaneous injections of synthetic leukotrienes in human skin were demonstrated by Soter et al. (See Soter et al., J. Clin Invest Dermatol, 80, 115-119 (1983).] Cutaneous vasodilation with edema formation and a neutrophil infiltrate were induced. Leukotriene synthesis inhibitors or leukotriene antagonists can also be useful in the treatment of ocular or dermatological diseases such as allergic conjunctivitis, uveitis, allergic dermatitis or psoriasis.

Another area of utility for leukotriene antagonists is in the treatment of cardiovascular diseases. Since peptide leukotrienes are potent coronary vasoconstrictors, they are implicated in a variety of cardiac disorders including arrhythmias, conduction blocks and cardiac depression. Synthetic leukotrienes have been shown to be powerful myocardial depressants, their effects consisting of a decrease in contractile force and coronary flow. The cardiac effects of $LTC_4$ and $LTD_4$ have been shown to be antagonized by a specific leukotriene antagonist, thus suggesting usefulness of leukotriene antagonists in the areas of myocardial depression and cardiac anaphylaxis. [See Burke, J.A., et al., J. Pharmacology and Experimental Therapeutics, 221, 1, 235-241 (1982).]

$LTC_4$ and $LTD_4$ have been measured in the body fluids of rats in endotoxic shock, but are rapidly cleared from the blood into the bile. Thus leukotrienes are formed in ischemia and shock. Specific inhibitors of leukotriene biosynthesis reduce the level of leukotrienes and therefore reduce manifestations of traumatic shock, endotoxic shock, and acute myocardial ischemia. Leukotriene receptor antagonists have also been shown to reduce manifestations of endotoxic shock and to reduce extension of infarct size. Administration of peptide leukotrienes has been shown to produce significant ischemia or shock. [See Lefer, A.M., Biochemical Pharmacology, 35, 2, 123-127 (1986).] Thus further areas of utility for leukotriene antagonists can be the treatment of myocardial ischemia, acute myocardial infarction, salvage of ischemic myocardium, angina, cardiac arrhythmias, shock and atherosclerosis.

Leukotriene antagonists can also be useful in the area of renal ischemia or renal failure. Badr et al. have shown that $LTC_4$ produces significant elevation of mean arterial pressure and reductions in cardiac output and renal blood flow, and that such effects can be abolished by a specific leukotriene antagonist. [See Badr, K.F. et al., Circulation Research, 54, 5, 492-499 (1984). Leukotrienes have also been shown to have a role in endotoxin-induced renal failure and the effects of the leukotrienes selectively antagonized in this model of renal injury. [See Badr, K.F., et al., Kidney International, 30, 474-480 (1986).] $LTD_4$ has been shown to produce local glomerular constrictor actions which are prevented by treatment with a leukotriene antagonist. [See Badr, K.F. et al., Kidney International, 29, 1, 328 (1986). $LTC_4$ has been demonstrated to contract rat glomerular mesangial cells in culture and thereby effect intraglomerular actions to reduce filtration surface area. [See Dunn, M.J. et al., Kidney International, 27, 1, 256 (1985). Thus another area of utility for leukotriene antagonists can be in the treatment of glomerulonephritis.

Leukotrienes have also been indicated in the area of transplant rejection. An increase in cardiac and renal allograft survival in the presence of a leukotriene receptor antagonist was documented by Foegh et al. [See Foegh, M.L. et al. Advances in Prostaglandin, Thromboxane, and Leukotriene Research, 13, 209-217 (1985).] Rejection of rat renal allografts was shown to produce increased amounts of $LTC_4$. [See Coffman, T.M. et al., Kidney International, 29, 1, 332 (1986).

A further area of utility for leukotriene antagonists can be in treatment of tissue trama, burns, or fractures. A significant increase in the production of cysteinyl leukotrienes was shown after mechanical or thermal trauma sufficient to induce tissue edema and circulatory and respiratory dysfunction. [See Denzlinger, C. et al., Science, 230, 330-332 (1985).]

Leukotrienes have also been shown to have a role in acute inflammatory actions. $LTC_4$ and $LTD_4$ have potent effects on vascular caliber and permeability and $LTB_4$ increases leukocyte adhesion to the endothelium. The arteriolar constriction, plasma leakage, and leukocyte adhesion bear close resemblence to the early events in acute inflammatory reactions. [See Dahlen, S.E. et al., Proc. Natl. Acad. Sci. USA, 78, 6, 3887-3891 (1981).] Mediation of local homeostasis and inflammation by leukotrienes and other mast cell-

dependent compounds was also investigated by Lewis et al. [See Lewis, R.A. et al., Nature, 293, 103-108 (1981). Leukotriene antagonists can therefore be useful in the treatment of inflammatory diseases including rheumatoid arthritis and gout.

Cysteinyl leukotrienes have also been shown to undergo enterohepatic circulation, and thus are indicated in the area of inflammatory liver disease. [See Denzlinger, C. et al., Prostaglandins Leukotrienes and Medicine, 21, 321-322 (1986).] Leukotrienes can also be important mediators of inflammation in inflammatory bowel disease. [See Peskar, B.M. et al., Agents and Actions, 18, 381-383 (1986).] Leukotriene antagonists thus can be useful in the treatment of inflammatory liver and bowel disease.

Leukotrienes have been shown to modulate IL-1 production by human monocytes. [See Rola-Pleszczynski, M. et al., J. of Immun., 135, 6, 3958-3961 (1985). This suggests that leukotriene antagonists may play a role in IL-1 mediated functions of monocytes in inflammation and immune reactions.

$LTA_4$ has been shown to be a factor in inducing carcinogenic tumors and is considered a link between acute immunologic defense reactions and carcinogenesis. Leukotriene antagonists can therefore possibly have utility in treatment of some types of carcinogenic tumors. [See Wischnewsky, G.G. et al. Anticancer Res. 5, 6, 639 (1985).]

Leukotrienes have been implicated in gastric cytodestruction and gastric ulcers. Damage of gastro intestinal mucosa because of potent vasoconstriction and stasis of blood flow is correlated with increased levels of $LTC_4$. Functional antagonism of leukotriene effects may represent an alternative in treatment of mucosal injury. [See Dreyling, K.W. et al., British J. Pharmacology, 88, 236P (1986), and Peskar, B.M. et al. Prostaglandins, 31, 2, 283-293 (1986).] A leukotriene antagonist has been shown to protect against stress-induced gastric ulcers in rats. [See Ogle, C.W. et al., IRCS Med. Sci., 14, 114-115 (1986).]

Other areas in which leukotriene antagonists can have utility because leukotrienes are indicated as mediators include prevention of premature labor [See Clayton, J.K. et al., Proceedings of the BPS, 573P, 17-19 Dec. 1984]; treatment of migraine headaches [See Gazzaniga, P.P. et al., Abstracts Int'l Conf. on Prostaglandins and Related Comp., 121, Florence, Italy (June 1986)]; and treatment of gallstones [See Doty, J.E. et al., Amer. J. of Surgery, 145, 54-61 (1983) and Marom, Z. et al., Amer. Rev. Respir. Dis., 126, 449-451 (1982).

By antagonizing the effects of $LTC_4$, $LTD_4$ and $LTE_4$ or other pharmacologically active mediators at the end organ, for example airway smooth muscle, the compounds and pharmaceutical compositions of the instant invention are valuable in the treatment of diseases in subjects, including human or animals, in which leukotrienes are a key factor.

DETAILED DESCRIPTION OF THE INVENTION

The compounds of this invention are represented by the following general structural formula (I)

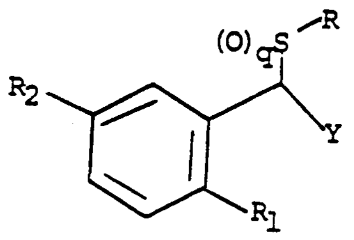

(I)

wherein

q is 1 or 2;

$R_1$ is $C_8$ to $C_{13}$ alkyl, $C_7$ to $C_{12}$ alkoxy, $C_{10}$ to $C_{12}$ 1-alkynyl, 10-undecynyloxy, 11-dodecynyl, phenyl-$C_4$ to $C_{10}$ alkyl, phenyl-$C_3$ to $C_9$ alkoxy with the phenyl optionally mono substituted with bromo, chloro, trifluoromethyl, $C_1$ to $C_4$ alkoxy, methylthio or trifluoromethylthio, furyl-$C_4$ to $C_{10}$ alkyl, trifluoromethyl-$C_7$ to $C_{12}$ alkyl or cyclohexyl-$C_4$ to $C_{10}$ alkyl;

$R_2$ is hydrogen, bromo, chloro, methyl, trifluoromethyl, hydroxy, $C_1$ to $C_4$ alkoxy or nitro; or $R_1$ is hydrogen and $R_2$ is $C_8$ to $C_{13}$ alkyl, $C_7$ to $C_{12}$ alkoxy, $C_{10}$ to $C_{12}$ 1-alkynyl, 10-undecynyloxy, 11-dodecynyl, phenyl-$C_4$ to $C_{10}$ alkyl, phenyl-$C_3$ to $C_9$ alkoxy with the phenyl optionally mono substituted with bromo, chloro, trifluoromethyl, $C_1$ to $C_4$ alkoxy, methylthio or trifluoromethylthio, furyl-$C_4$ to $C_{10}$ alkyl, trifluoromethyl-$C_7$ to $C_{12}$ alkyl or cyclohexyl-$C_4$ to $C_{10}$ alkyl;

Y is $COR_3$,

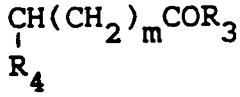,

or $(CH_2)_{0-1}$-C-tetrazolyl;

$R_3$ is hydroxy, amino, or $C_1$ to $C_6$ alkoxy;

$R_4$ is hydrogen, methyl, $C_1$ to $C_4$ alkoxy, fluoro or hydroxy;

m is 0, 1, and 2;

R is

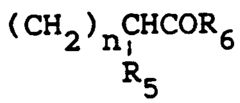,

$CH(CO_2H)CH_2CO_2H$, $CH_2CH_2Z$ or

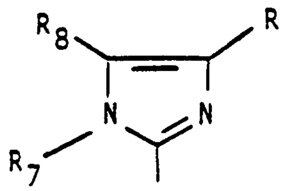;

n is 0 to 6;

$R_5$ is hydrogen, amino, or $NHCOCH_2CH_2CH(NH_2)CO_2H$;

$R_6$ is hydroxy, amino, $NHCH_2CO_2H$, or $C_1$ to $C_6$ alkoxy;

Z is $SO_3H$, $SO_2NH_2$ or CH;

$R_7$ is hydrogen, $C_1$ to $C_4$ alkyl or $C_3$ to $C_4$ alkenyl;

$R_8$ is hydrogen, $C_1$ to $C_4$ alkyl, carboxyl or carboxamido, or $(CH_2)_pCO_2R_{12}$, wherein p is 1 or 2, $R_{12}$ is $C_1$ to $C_6$ alkyl, or hydrogen,

when $R_7$ and $R_9$ are hydrogen or $C_1$ to $C_4$ alkyl; and

$R_9$ is hydrogen, $C_1$ to $C_4$ alkyl or $CH_2CO_2R_{13}$ wherein $R_{13}$ is $C_1$ to $C_6$ alkyl, or hydrogen, with the proviso that when n is 0, $R_5$ is hydrogen and further that $R_7$, $R_8$ and $R_9$ are not all hydrogen; or a pharmaceutically acceptable salt thereof.

The ester and diester compounds of Formula (I) are subject to the further proviso that $R_3$ and $R_6$ are not both hydroxy or $R_3$ is not hydroxy if both $R_{12}$ and $R_{13}$ are hydrogen.

A particular class of compounds of this invention are the substituted alkanoic acid analogs of formula (I) represented by the structural formula (II)

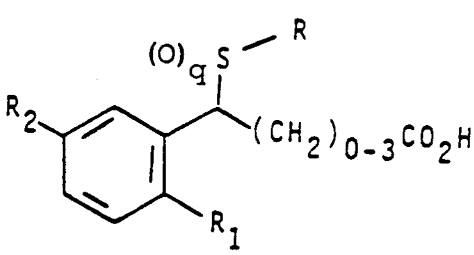

(II)

wherein q, $R_1$, $R_2$ and R are described above.

Particular members of this class of compounds are those represented by the structural formula (II) wherein R is $(CH_2)_{1-3}CO_2H$ or

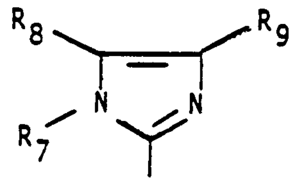

and $R_1$, $R_2$, $R_7$, $R_8$ and $R_9$ are described above.

A subgeneric class of these compounds are the diacid derivatives represented by the following general structural formula (III)

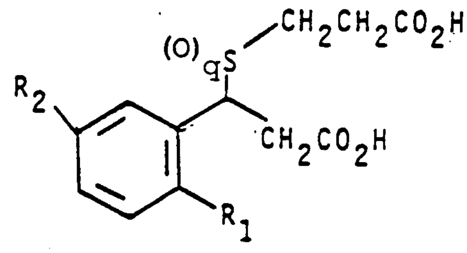

(III)

wherein q, $R_1$ and $R_2$ are described above, and particularly where $R_1$ is phenylalkyl.

A second subgeneric class of compounds of formula (II) are the diacid derivatives represented by the following structural formula (IV)

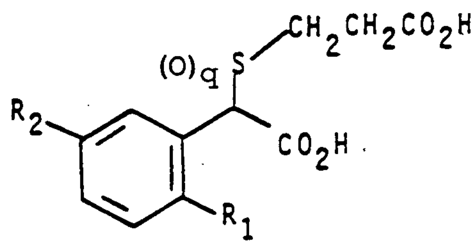

(IV)

wherein q, $R_1$ and $R_2$ are described above, and particularly where $R_1$ is phenylalkyl.

A third subgeneric class of compounds of formula (II) are the heterocyclic derivatives represented by the following general structural formula (V)

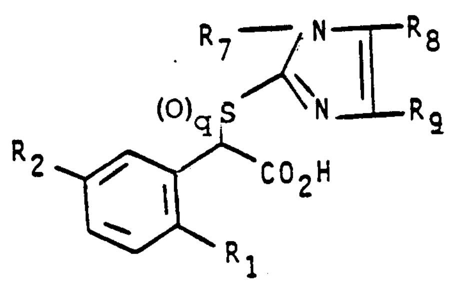

(V)

wherein q, $R_1$, $R_2$, $R_7$, $R_8$ and $R_9$ are described above.

A further particular class of compounds of this invention are the hydroxy substituted alkanoic acid analogs of formula (I) represented by the structural formula (VI)

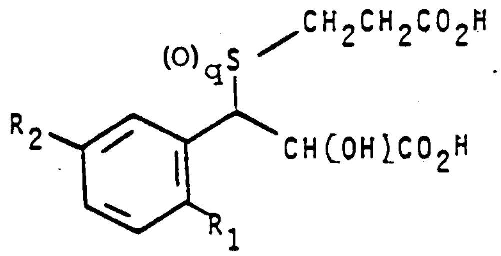

(VI)

wherein q, $R_1$ and $R_2$ are described above, and particularly where $R_1$ is phenylalkyl.

The compounds of the formula (VI) are exemplified by the following compounds:

(1) 2(S)-hydroxy-3(R)-(2-carboxyethylsulfinyl)-3-[2-(8-phenyloctyl)phenyl]propionic acid; and

(2) 2(S)-hydroxy-3(R)-(2-carboxyethylsulfonyl)-3-[2-(8-phenyloctyl)phenyl]propionic acid.

A further class of compounds of this invention are the tetrazolyl substituted analogs of formula (I) represented by the structural formula (VII)

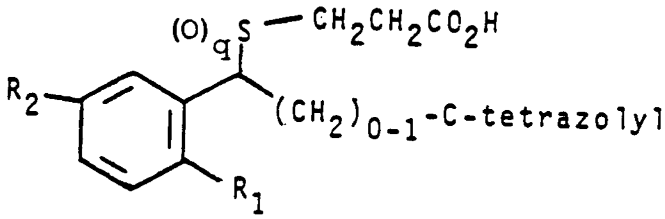

(VII)

wherein q, $R_1$ and $R_2$ are described above.

Some of the compounds of the formula (I) contain two asymmetric centers, such as when $R_4$ is methyl, methoxy, fluoro or hydroxy, or R is $CH(CO_2H)CH_2CO_2H$. This leads to the possibility of four stereoisomers for each such compound. In practice, these compounds are prepared as a mixture of two stereoisomers. Resolution procedures employing, for example, optically active amines furnish the separated enantiomers.

The compounds of the present invention, depending on their structure, are capable of forming salts with pharmaceutically acceptable acids and bases, according to procedures well known in the art. Such acceptable acids include inorganic and organic acids, such as hydrochloric, sulfuric, methanesulfonic, benzenesulfonic, p-toluenesulfonic and acetic acid. Such acceptable bases include organic and inorganic bases, such as ammonia, arginine, organic amines, alkali metal bases and alkaline earth metal bases. Of particular utility are the dipotassium, disodium, dimagnesium, diammonium, and dicalcium salts of the diacid compounds of formula (I).

The compounds of the formula (I) wherein Y is $CO_2H$ are conveniently prepared from an aldehyde precursor of the following structural formula (VIII)

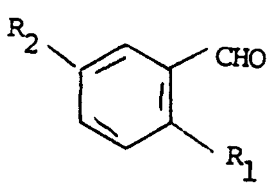

(VIII)

wherein $R_1$ and $R_2$ are described above. A compound of formula (VIII) is treated with trimethylsilyl cyanide in the presence of zinc iodide at low temperatures in an inert solvent to form the trimethylsilyl-protected cyanohydrin. Treatment of this with gaseous hydrogen chloride in methanol provides the methyl 2-hydroxyacetate derivative which is converted to the 2-chloroacetate with thionyl chloride. This valuable intermediate is then reacted with a substituted thiol selected to give, after removal of ester protective groups, a sulfide analogue of formula (I).

The compounds of the formula (I) wherein Y is $CH_2CO_2H$ are prepared by reacting the appropriate aldehyde of the formula (VIII) and an esterified bromoacetate, conveniently t-butyl bromoacetate, with a mixture of diethyl aluminum chloride, zinc dust and a catalytic amount of cuprous bromide at low temperatures in an inert solvent to give the esterified 3-hydroxypropionate derivative which is reacted

directly with a substituted thiol in trifluoroacetic acid. Alternatively, a mixture of trimethyl borate and zinc in tetrahydrofuran may be used to prepare the 3-hydroxypropionate derivative. By employing an esterified 2-bromopropionate in the above reaction with an aldehyde (VIII), the sulfide compounds wherein Y is $CH(CH_3)CO_2H$ are obtained.

To prepare the desired compounds of formula (I) wherein q is 1 or 2, the appropriate thio product is conveniently oxidized with sodium periodate or metachloroperbenzoic acid to obtain the sulfoxide or sulfone product.

The aldehydes of the formula (VIII) are known or readily prepared utilizing the general procedures described as follows.

The aldehyde precursors to the compounds of the formula (I) wherein $R_1$ is, for example, an alkyl radical containing 8 to 13 carbon atoms are prepared from the appropriate 2-methoxyphenyl-4,4-dimethyloxazoline [see Meyers et al. J. Org. Chem., 43 1372 (1978)].

The aldehyde precursors of the compounds of the formula (I) wherein $R_1$ is, for example, an alkoxy radical containing 7 to 12 carbon atoms are prepared by the 0-alkylation of the appropriate 2-hydroxybenzaldehyde with the corresponding alkylating agent.

The aldehyde precursors to the compounds of the formula (I) wherein $R_1$ is a 1-alkynyl radical containing 10 to 12 carbon atoms are prepared by coupling a 2-halobenzaldehyde with the appropriate 1-alkyne in the presence of cuprous iodide and $(PO_3)_2PdCl_2$. [See Hagihara, et al. Synthesis, 627, (1980)]. The catalytic hydrogenation of these alkynyl containing precursors under standard conditions affords the aldehyde precursors of the compounds of the formula (I) wherein $R_1$ is an alkyl or phenylalkyl radical.

Alternatively, the compounds of the formula (I) wherein Y is $CH_2CO_2H$ are prepared from a propenoate precursor of the following structural formula (IX)

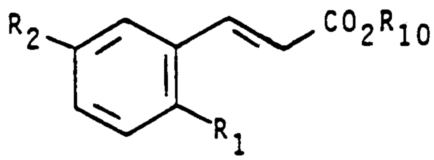

(IX)

wherein $R_1$ and $R_2$ are described above, and $R_{10}$ is an ester protective group, such as t-butyl. A compound of formula (IX) is reacted with a mixture of alkali metal alkoxide, such as sodium methoxide, and substituted thiol to give, after removal of the ester protective group, sulfide analogs of formula (I). These are oxidized as previously described to obtain the desired products of formula (I).

The propenoate precursors of formula (IX) are prepared from the corresponding aldehydes of formula (VIII) by general procedures such as reaction with an alkyl (triphenylphosphoranylidene) acetate or by conversion of the aldehyde to a 3-hydroxypropionate derivative, as described above, followed by an elimination reaction to form the double bond. Additionally, the propionate precursor is obtained from a 3-methanesulfonyloxypropionate derivative by treatment with triethylamine.

The compounds of the formula (I) wherein Y is $CH(OH)(CH_2)_mCO_2H$ are prepared from an epoxide precursor of the following structural formula (X)

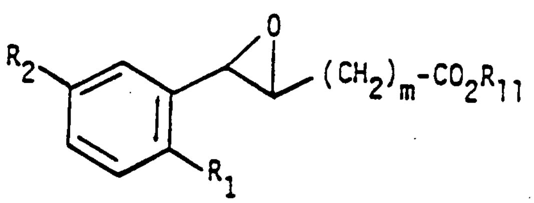

(X)

wherein $R_1$, $R_2$ and m are described above, and $R_{11}$ is lower alkyl, such as methyl or ethyl. A compound of formula (X) is reacted in an inert solvent with triethylamine and a substituted thiol selected to give, after removal of ester protective groups and oxidation, a product of formula (I).

The epoxide precursors of formula (X) where m is 2 are prepared by reaction of the Grignard derivative of a bromobenzene compound of the formula (XI)

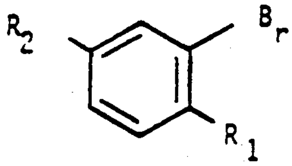

(XI)

with acrolein to give the corresponding enol derivative which is treated with a trialkylorthoacetate, followed by epoxidation using metachloroperbenzoic acid.

The epoxide precursors of formula (X) where m is 0 are prepared by reaction of an aldehyde of the formula (VIII) with a lower alkyl chloroacetate and an alkali metal alkoxide, such as sodium methoxide.

Alternatively, the compounds of the formula (I) wherein Y is $CH(OH)COR_3$ are prepared from a propenoate precursor of formula (IX) wherein $R_{10}$ is lower alkyl.

The compounds of the formula (I) wherein Y is $(CH_2)_3CO_2H$ are prepared from a tetrahydro-4H-pyran-2-one precursor of the following structural formula (XII)

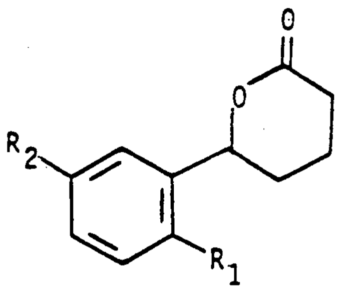

(XII)

wherein $R_1$ and $R_2$ are described above. A compound of formula (XII) is reacted with a mixture of zinc iodide and a substituted thiol in an inert solvent or with a substituted thiol in trifluoroacetic acid to give, after removal of any ester protective group and oxidation, a product of formula (I).

The tetrahydro-4H-pyran-2-one precursors of formula (XII) are prepared by reaction of the Grignard derivative of the bromobenzene compound of formula (XI) with chloro titanium tri-isopropoxide followed by reaction with 5-oxovalerate alkyl ester.

The 2-thioimidazole precursors necessary to prepare the R-heterocyclic derivatives of formula (I) are known compounds or are conveniently prepared employing standard chemical reactions. Preferably these reactants bearing a carboxyl or carboxymethyl substituent as set forth in $R_8$ and $R_9$ above are employed as the corresponding carboalkoxy derivatives wherein the alkoxy radical contains from one to six carbon atoms. When present, the alkoxy substitutent is subsequently hydrolyzed to give the free carboxyl or carboxymethyl substituted products.

Appropriate modifications of the general processes disclosed furnish the various compounds defined by formula (I).

The leukotriene antagonist activity of the compounds of this invention is measured by the ability of the compounds to inhibit the leukotriene induced contraction of guinea pig tracheal tissues in vitro and to inhibit leukotriene induced bronchoconstriction in guinea pigs in vivo. The following methodologies were employed:

In vitro: Guinea pig (adult male albino Hartley strain) tracheal spiral strips of approximate dimensions 2 to 3 mm cross-sectional width and 3.5 cm length were bathed in modified Krebs buffer in jacketed 10 ml tissue bath and continously aerated with 95% $O_2$/5% $CO_2$. The tissues were connected via silk suture to force displacement transducers for recording isometric tension. The tissues were equilibrated for 1 hr., pretreated for 15 minutes with meclofenamic acid (1$\mu$M) to remove intrinsic prostaglandin responses, and then pretreated for an additional 30 minutes with either the test compound or vehicle control. A cumulative concentration-response curve for $LTD_4$ on triplicate tissues was generated by successive increases in the bath concentration of the $LTD_4$. In order to minimize intertissue variability, the contractions elicited by $LTD_4$ were standardized as a percentage of the maximum response obtained to a reference agonist, carbachol (10$\mu$M).

Calculations: The averages of the triplicate $LTD_4$ concentration-response curves both in the presence and absence of the test compound were plotted on log graph paper. The concentration of $LTD_4$ needed to elicit 30% of the contraction elicited by carbachol was measured and defined as the $EC_{30}$. The -log $K_B$ value for the test compound was determined by the following equations:

$$1.\quad \frac{EC_{30}\ \text{(presence of test compound)}}{EC_{30}\ \text{(presence of vehicle control)}} = \text{dose ratio} = X$$

$$2.\quad K_B = \text{concentration of test compound}/(X-1)$$

In vivo: Anesthetized, spontaneously breathing guinea pigs (Adult male albino Hartley strain) were monitored on a Buxco pulmonary mechanics computer. Changes in airway resistance ($R_L$) were calculated by the computer on a breath-by-breath basis at isovolumic points from signals measuring airflow and transpulmonary pressure using differential pressure transducers. Animals were pretreated with 1 mg/kg of propranolol, iv, followed by 100 puffs of an aqueous solution of test compound or vehicle control by aerosol via a Monaghan nebulizer. $LTD_4$ was then aerosolized into the airway. The bronchoconstriction produced was reflected by % changes in airways resistance relative to the baseline values obtained prior to injection of the test compound or vehicle control. Each guinea pig received either vehicle control or test compound. Calculations: The average of 3 - 6 animals per treatment was calculated using the % changes in the pulmonary parameters for control and test compound-treated animals. The average $ inhibition by the test compound was calculated from the following equation:

$$\%\ \text{Inhibition} = \frac{\underset{\text{(vehicle control)}}{R_L} - \underset{\text{(test compound)}}{R_L}}{\underset{\text{(vehicle control)}}{R_L}} \times 100$$

The compounds of this invention possess biosignificant antagonist activity against leukotrienes, primarily leukotriene $D_4$. The antagonist activity of representative compounds of this invention is tabulated below (other data appears in the preparative examples). The -log $K_B$ values and the $R_L$ values were calculated from the above test protocols. Where compounds were tested more than once, the -log $K_B$ values given herein represent the current average data.

Compounds of the Formula (VI)

| $R_1$ | $R_2$ | q | In Vitro -Log $K_B$ |
|---|---|---|---|
| $-C_8H_{16}$phenyl | H | 1 | 7.6 |
| $-C_8H_{16}$ phenyl | H | 2 | 7.7 |

The specificity of the antagonist activity of a number of the compounds of this invention is demonstrated by relatively low levels of antagonism toward agonists such as potassium chloride, carbachol, histamine and $PGF_2$ .

Pharmaceutical compositions of the present invention comprise a pharmaceutical carrier or diluent and an amount of a compound of the formula (I) or a pharmaceutically acceptable salt, such as an alkali metal salt thereof, sufficient to produce the inhibition of the effects of leukotrienes.

When the pharmaceutical composition is employed in the form of a solution or suspension, examples of appropriate pharmaceutical carriers or diluents include: for aqueous systems, water; for non-aqueous systems, ethanol, glycerin, propylene glycol, corn oil, cottonseed oil, peanut oil, sesame oil, liquid parafins and mixtures thereof with water; for solid systems, lactose, kaolin and mannitol; and for aerosol systems, dichlorodifluoromethane, chlorotrifluoroethane and compressed carbon dioxide. Also, in addition to the pharmaceutical carrier or diluent, the instant compositions may include other ingredients such as stabilizers, antioxidants, preservatives, lubricants, suspending agents, viscosity modifiers and the like, provided that the additional ingredients do not have a detrimental effect on the therapeutic action of the instant compositions.

The nature of the composition and the pharmaceutical carrier or diluent will, of course, depend upon the intended route of administration, i.e. parenterally, topically, orally or by inhalation.

In general, particularly for the prophylactic treatment of asthma, the compositions will be in a form suitable for administration by inhalation. Thus the compositions will comprise a suspension or solution of the

active ingredient in water for administration by means of a conventional nebulizer. Alternatively the compositions will comprise a suspension or solution of the active ingredient in a conventional liquified propellant or compressed gas to be administered from a pressurized aerosol container. The compositions may also comprise the solid active ingredient diluted with a solid diluent for administration from a powder inhalation device. In the above compositions, the amount of carrier or diluent will vary but preferably will be the major proportion of a suspension or solution of the active ingredient. When the diluent is a solid it may be present in lesser, equal or greater amounts than the solid active ingredient.

For parenteral administration the pharmaceutical composition will be in the form of a sterile injectable liquid such as an ampul or an aqueous or nonaqueous liquid suspension.

For topical administration the pharmaceutical composition will be in the form of a cream, ointment, liniment, lotion, pastes, and drops suitable for administration to the eye, ear, or nose.

For oral administration the pharmaceutical composition will be in the form of a tablet, capsule, powder, pellet, atroche, lozenge, syrup, liquid, or emulsion.

Usually a compound of formula I is administered to a subject in a composition comprising a nontoxic amount sufficient to produce an inhibition of the symptoms of a disease in which leukotrienes are a factor. When employed in this manner, the dosage of the composition is selected from the range of from 350 mg. to 1000 mg. of active ingredient for each administration. For convenience, equal doses will be administered 1 to 5 times daily with the daily dosage regimen being selected from about 350 mg. to about 5000 mg.

The pharmaceutical preparations thus described are made following the conventional techniques of the pharmaceutical chemist as appropriate to the desired end product.

Included within the scope of this disclosure is the method of treating a disease, pulmonary or non-pulmonary, in which leukotrienes are a factor which comprises administering to a subject a therapeutically effective amount of a compound of formula I, preferably in the form of a pharmaceutical composition. For example, inhibiting the symptoms of an allergic response resulting from a mediator release by administration of an effective amount of a compound of formula I is included within the scope of this disclosure. The administration may be carried out in dosage units at suitable intervals or in single doses as needed. Usually this method will be practiced when relief of symptoms is specifically required. However, the method is also usefully carried out as continuous or prophylactic treatment. It is within the skill of the art to determine by routine experimentation the effective dosage to be administered from the dose range set forth above, taking into consideration such factors as the degree of severity of the condition or disease being treated, and so forth.

Compounds of this invention, alone and in combination with a histamine $H_1$-receptor antagonist, inhibit antigen-induced contraction of isolated, sensitized guinea pig trachea (a model of respiratory anaphylaxis). Exemplary of compounds of this invention are 2(S)-hydroxy-3(R)-(2-carboxyethylsulfinyl)-3-[2-(8-phenyloctyl)phenyl]-propanoic acid. Exemplary of histamine $H_1$-receptor antagonists are mepyramine, chlorpheniramine, and 2-[4-(5-bromo-3-methyl-pyrid-2-yl)butylamino]-5-[(6-methylpyrid-3-yl)methyl]-4-pyrimidone and other known $H_1$-receptor antagonists.

Pharmaceutical compositions, as described hereinabove, of the present invention also comprise a pharmaceutical carrier or diluent and a combination of a compound of the formula (I) or a pharmaceutically acceptable salt thereof, and an histamine $H_1$-receptor antagonist in amounts sufficient to inhibit antigen-induced respiratory anaphylaxis. The above-defined dosage of a compound of formula I is conveniently employed for this purpose and the known effective dosage for the histamine $H_1$-receptor antagonist. The methods of administration described above for the single active ingredient can similarly be employed for the combination with a histamine $H_1$-receptor antagonist.

The following examples illustrate the preparation of the compounds of this invention and their incorporation into pharmaceutical compositions and as such are not to be considered as limiting the invention set forth in the claims appended hereto.

EXAMPLE 1

Preparation of 2-Hydroxy-3-(2-carboxyethylthio)-3-[2-(8-phenyloctyl)phenyl]propionic acid

(a) 2-(8-Phenyloctyl)benzaldehyde

A solution of 8-phenyloctanoic acid (19.8 mmol) in sieve dried tetrahydrofuran (5 ml) was reduced with diborane in tetrahydrofuran (30 ml, 29.1 mmol) at 0 °C for 4 hours to give 8-phenyloctanol. To an ice cold solution of the octanol (ca. 19.8 mmol) and carbon tetrabromide (21.98 mmol) in methylene chloride (50 ml) was added triphenylphosphine (22.30 mmol) in methylene chloride (50 ml) and the resulting solution was

stirred for 2.5 hours. The volatiles were evaporated and the residue was taken up in ether (100 ml), cooled in ice, and filtered. The filtrate was evaporated and distilled to afford 8-phenyloctyl bromide as an oil.

To 8-phenyloctylmagnesium bromide (from 24.25 mmol of 8-phenyloctyl bromide and 21.27 mmol of magnesium) in distilled tetrahydrofuran (40 ml) was added 2-(2-methoxyphenyl)-4,4-dimethyloxazoline (17.10 mmol) [A.I. Meyers et al., J. Org. Chem., 43, 1372 (1978)] in tetrahydrofuran (20 ml). After stirring for 24 hours, the reaction mixture was worked up to yield 2-[2-(8-phenyloctyl)phenyl]-4,4-dimethyloxazoline as an oil. A solution of the oxazoline (11.58 mmol) in methyl iodide (20 ml) was refluxed under argon for 18 hours. Removal of the volatiles afforded the corresponding 3,4,4-trimethyl- oxazolinium iodide as a white solid (mp 76.5-78° C).

To an ice cold solution of the iodide (9.46 mmol) in methanol (35 ml) was added in portions sodium borohydride (9.20 mmol). The reaction mixture was allowed to stir for 30 minutes and was then quenched with 5 percent sodium hydroxide (50 ml). The reaction mixture was extracted with diethyl ether (2 x 50 ml) and the extract was washed with brine (50 ml) and dried over anhydrous magnesium sulfate and filtered. Evaporation of the filtrate afforded an oil which was dissolved in acetone (50 ml) and 3N hydrochloric acid (10 ml) was added. The mixture was flushed with argon and stirred for 16 hours at ambient temperature. The volatiles were removed under vacuum and the residue partitioned between diethyl ether (50 ml) and water (50 ml). The aqueous phase was extracted with more diethyl ether (50 ml). The combined organic phase was washed with brine (50 ml) and dried over anhydrous magnesium sulfate. Evaporation of the organic phase yielded an oil which was purified by flash chromatography over silica gel with 2 percent ethyl acetate in hexane as eluant to afford the desired product as a colorless oil.

Analysis for $C_{21}H_{26}O$: Calculated: C, 85.67; H, 8.90. Found: C, 85.12, 85.22; H, 8.94, 8.96.

(b) Alternative preparation of 2-(8-phenyloctyl)-benzaldehyde

A solution of 5-hexynyl alcohol (102 mmol) in pyridine (150 ml), under argon, was cooled to 0° C and p-toluenesulfonyl chloride (204 mmol) was added. The reaction mixture was kept at about 4° C for 18 hours, poured into ice-water and then taken up in ether. The ether extract was washed with cold 10% hydrochloric acid, water and brine. The organic layer was dried and concentrated in vacuo to give 5-hexynyl p-toluenesulfonate. A solution of phenylacetylene (97 mmol) in tetrahydrofuran (200 ml) containing a trace of triphenylmethane was cooled to 0° C and then n-butyl lithium (37.3 ml of 2.6 mol in hexane) was added dropwise. The resulting solution was stirred at 0° C for 10 minutes and hexamethylphosphoramide (21 ml) was added dropwise. After stirring for 10 minutes a solution of 5-hexynyl p-toluenesulfonate (97.1 mmol) in tetrahydrofuran (200 ml) was added. The reaction mixture was stirred at room temperature for 18 hours, diluted with ether and the organic layer was washed with water and brine. The dried organic solution was concentrated and the product was purified by flash chromatography to give 1-phenylocta-1,7-diyne. A mixture of this compound (43 mmol), 2-bromobenzaldehyde (35.8 mmol), cuprous iodide (0.5 mmol) and bis(triphenylphosphine) palladium (II) chloride (0.7 mmol) in triethylamine (100 ml) was heated in an oil bath (95° C) for one hour. The reaction mixture was cooled to 0° C, filtered and the filtrate was concentrated. The residue was dissolved in ether, washed with 10% hydrochloric acid, water and brine. The organic layer was dried and concentrated to give a product which was purified by flash chromatography to yield 2-(8-phenyl-1,7-octadiynyl)benzaldehyde. A solution of this compound (24.1 mmol) in ethyl acetate (100 ml) and 10% palladium on charcoal (1 g) was hydrogenated (40 psi of hydrogen) at room temperature for 15 minutes. The catalyst was filtered off and the filtrate concentrated to give the 2-(8-phenyloctyl)benzaldehyde.

(c) Methyl trans-3-[2-(8-Phenyloctyl)phenyl]-2,3-epoxypropionate

The compound of Example 1(a) or (b) (2.94 g, 10 mmol) was dissolved in diethyl ether (25 ml) and the solution was stirred under argon at 0° C. Methyl chloroacetate (1.32 ml, 15 mmol) was added, followed by the addition of sodium methoxide (810 mg, 15 mmol). The mixture was stirred for 2.5 hours at ice bath temperature. A small quantity of water was added, the ether phase separated, dried over anhydrous sodium sulfate, filtered and evaporated. The residue was flash chromatographed on 80 grams of silica gel eluted with 5-30% ethyl acetate/hexane to give the product.

(d) Methyl 3-(2-Carbomethoxyethylthio)-3-[2-(8-phenyloctyl)phenyl]-2-hydroxypropionate

The compound of Example 1(c) (1.2 g, 3.28 mmol) was dissolved in methanol (20 ml) containint 2% triethylamine and stirred under argon at room temperature. Methyl 3-mercaptopropionate (0.623 ml, 5.45 mmoles) and triethylamine (1.45 ml, 9.84 mmol) were dissolved in methanol (15 ml) and added dropwise.

The mixture was stirred for 18 hours. The solvent was stripped and the residue eluted with 20% ethyl acetate/hexane to give a mixture of the desired product and its regioisomer, methyl 2-(2-carbomethoxyethylthio)-3-[2-(8-phenyloctyl)phenyl]-3- hydroxypropionate. The mixture was rechromatographed on 100g of neutral alumina to separate the desired product.

(e) Erythro-3-(2-carboxyethylthio)-3-[2-(8-phenyloctyl)phenyl]-2-hydroxypropionic acid)

The desired product of Example 1(d) (320 mg, 0.66 mmol) was dissolved in methanol (10 ml) and stirred under argon at ice bath temperature. A 1N solution of sodium hydroxide (2.5 ml, 2.5 mmol) was added dropwise, the ice bath removed, the mixture stirred at room temperature for 2.5 hours, and then cooled for 18 hours. After an additional 1 hour of stirring at room temperature, the methanol was stripped, the residue diluted with water and the pH adjusted to 3.5 with dilute hydrochloric acid. Extraction with ethyl acetate followed by drying over anhydrous sodium sulfate, filtration and evaporation gave the crude product which was flash chromatographed on 20 grams of silica gel eluted with 30:70:0.5 ethyl acetate:hexane:formic acid to give the free acid product.

(f) Resolution of 3-(2-carboxyethylthio)-3-[2-(8-phenyloctyl)phenyl]-2-hydroxypropionic acid

The racemic diacid of Example 1(e) (63.5 g, 0.138 mol) in 700 ml of isopropanol was treated with a solution of (R)-4-bromo-$\alpha$-phenethylamine (57.1 g, 0.286 mol) in 200 ml of isopropanol at 25 °C. The resulting solution was stirred for 3 hours, causing crystallization of the 2S,3R diamine salt. The suspension was cooled to 5 °C, filtered, and the salt recrystallized twice from ethanol to give 37.7 g (72%) of 2S,3R diamine salt, m.p. 146-147 °C; $[\alpha]^{24°C}$ = -15.8 ° (C = 1, $CH_3OH$).

The diamine salt (37.7 g, 0.0497 mol) was added in portions to 400 ml of cold 0.5N aqueous hydrochloric acid. The mixture was extracted with ethyl acetate, and the ethyl acetate solution washed three times with 0.5N hydrochloric acid. The ethyl acetate solution was washed with saturated sodium chloride solution, dried, and concentrated to give 19.5 g (97%) of the desired 2(S)-hydroxy-3(R)-(2-carboxyethylthio)-3-[2-(8-phenyloctyl)-phenyl]-propionic acid; $[\alpha]^{24°}$ = -40.8 ° (C = 1, $CHCl_3$).

(g) 2(S)-Hydroxy-3(R)-(2-carboxyethylsulfinyl)-3-[2-(8-phenyloctyl)phenyl]propionic acid

A suspension of the compound of Example 1(f) (870 mg, 1.9 mmole) in 15 ml of water was treated with NaOH (152 mg, 3.8 mmol), stirred until solution was complete, and cooled to 0 °. A solution of $NaIO_4$ (500 mg) in 8 ml of water was added. Stirring was continued at 0 ° for 45 minutes and at 23 ° for 1 hour. The reaction was acidified and extracted with ethyl acetate. The extracts were dried and the solvent evaporated. The residue was chromatographed over a silica gel column, and the product eluted with a mixture of ethyl acetate: hexane: acetic acid (80:20:3), and gave 470 g (52%). nmr ($CDCl_3/Me_2CO$) on the mixture of diastereomers: 8.72 (broad, 3H), 7.62 (d) and 7.96(d) (together 1H), 7.20 (s, 8H), 5.12 (m, 1H), 4.82 (m, 1H), 2.48 to 2.92 (m, 8H), 1.20 to 1.86 (m, 12H).

EXAMPLE 2

2(S)-Hydroxy-3(R)-(2-carboxyethylsulfonyl)-3-[2-(8-phenyloctyl)phenyl]propionic acid.

A solution of the compound of Example 1(f) (930 mg) in 75 ml of $CHCl_3$ was treated over 15 minutes with m-chloroperbenzoic acid (1 g). After 1-1/2 hours at 23 °, 3 ml of saturated aqueous $NaHSO_3$ was added. After 5 minutes 3N HCl was added. The organic layer was separated, washed with water, dried, and the solvent removed. The residue was chromatographed over a silica gel column, and eluted with a mixture of ethyl acetate: chloroform: acetic acid (50:50:1). After a forerun containing m-chlorobenzoic acid, the product was collected in the later fractions, and gave 740 g (75%). nmr ($CDCl_3/Me_2CO$) 10.0 (broad 3H), 8.10 (d, 1H), 7.03 to 7.33 (m, 8H), 5.29 (d, 1H), 5.03 (d, 1H), 3.24 to 3.60 (m, 2H) 2.46 to 2.92 (m, 6H), 1.16 to 1.88 (m, 12H).

**Claims**

**Claims for the following Contracting States : AT, BE, CH, DE, FR, GB, IT, LI, LU, NL, SE**

**1.** A compound represented by the following structural formula (I):

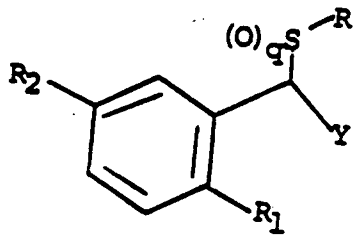

(I)

wherein

q is 1 or 2;

$R_1$ is $C_8$ to $C_{13}$ alkyl, $C_7$ to $C_{12}$ alkoxy, $C_{10}$ to $C_{12}$ 1-alkynyl, 10-undecynyloxy, 11-dodecynyl, phenyl-$C_4$ to $C_{10}$ alkyl, phenyl- $C_3$ to $C_9$ alkoxy with the phenyl optionally mono substituted with bromo, chloro, trifluoromethyl, $C_1$ to $C_4$ alkoxy, methylthio or trifluoromethylthio, furyl-$C_4$ to $C_{10}$ alkyl, trifluoromethyl-$C_7$ to $C_{12}$ alkyl or cyclohexyl-$C_4$ to $C_{10}$ alkyl;

$R_2$ is hydrogen, bromo, chloro, methyl, trifluoromethyl, hydroxy, $C_1$ to $C_4$ alkoxy or nitro; or $R_1$ is hydrogen and $R_2$ is $C_8$ to $C_{13}$ alkyl, $C_7$ to $C_{12}$ alkoxy, $C_{10}$ to $C_{12}$-1-alkynyl, 10-undecynyloxy, 11-dodecynyl, phenyl-$C_4$ to $C_{10}$-alkyl, phenyl-$C_3$ to $C_9$ alkoxy with the phenyl optionally mono substituted with bromo, chloro, trifluoromethyl, $C_1$ to $C_4$ alkoxy, methylthio or trifluoromethylthio, furyl-$C_4$ to $C_{10}$-alkyl, trifluoromethyl-$C_7$ to $C_{12}$ alkyl or cyclohexyl-$C_4$ to $C_{10}$ alkyl;

Y is $COR_3$,

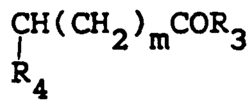

or $(CH_2)_{0-1}$-C-tetrazolyl;

$R_3$ is hydroxy or amino;

$R_4$ is hydrogen, methyl, $C_1$ to $C_4$ alkoxy, fluoro or hydroxy;

m is 0, 1, or 2;

R is

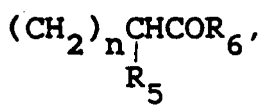

$CH(CO_2H)CH_2CO_2H$, $CH_2CH_2Z$ or

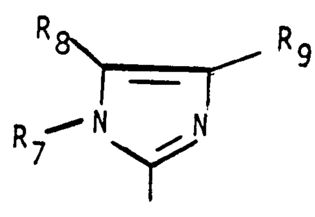

;

n is 0 to 6;

$R_5$ is hydrogen, amino, or $NHCOCH_2CH_2CH(NH_2)CO_2H$;

$R_6$ is hydroxy, amino or $NHCH_2CO_2H$;

Z is $SO_3H$, $SO_2NH_2$ or CN;

$R_7$ is hydrogen, $C_1$ to $C_4$ alkyl or $C_3$ to $C_4$ alkenyl;

$R_8$ is hydrogen, $C_1$ to $C_4$ alkyl, carboxyl or carboxamido, or $(CH_2)_pCO_2H$, wherein p is 1 or 2, when $R_7$ and $R_9$ are hydrogen or $C_1$ to $C_4$ alkyl; and

$R_9$ is hydrogen, $C_1$ to $C_4$ alkyl or $CH_2CO_2H$, with the proviso that when n is 0, $R_5$ is hydrogen and further that $R_7$, $R_8$ and $R_9$ are not all hydrogen; or a pharmaceutically acceptable salt thereof.

2. A compound of Claim 1 represented by the following structural formula (II):

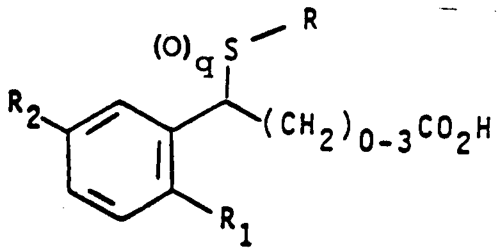

(II)

wherein R is $(CH_2)_{1-3}$ $CO_2H$ or

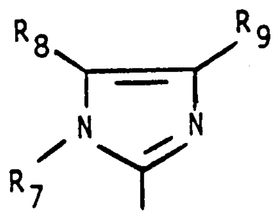

;

or the following structural formula (III):

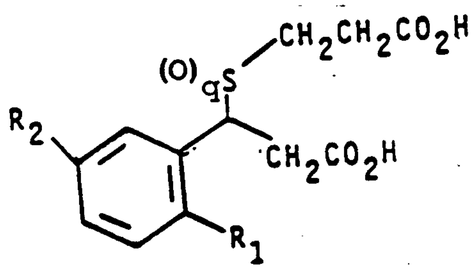

(III)

wherein $R_1$ is a phenyl-$C_4$ to $C_{10}$ alkyl radical; or the following structural formula (IV):

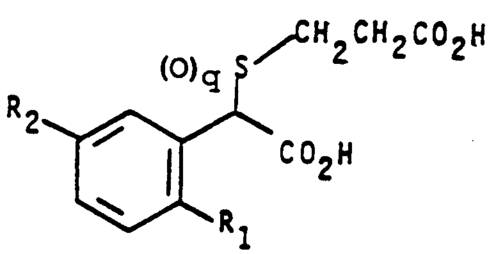

(IV)

wherein $R_1$ is a phenyl-$C_4$ to $C_{10}$ alkyl radical; or the following structural formula (V):

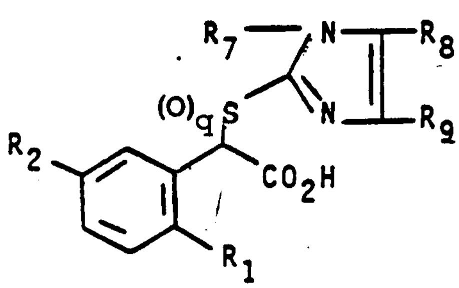

(V)

or the following structural formula (VI):

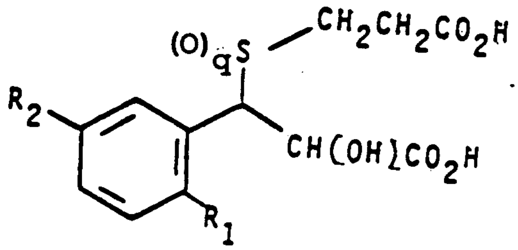

(VI)

wherein $R_1$ is a phenyl -$C_4$ to $C_{10}$ alkyl radical; or the following structural formula (VII):

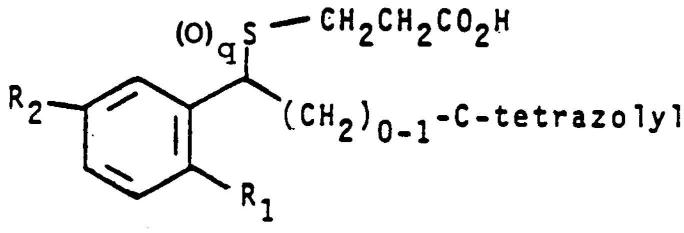

(VII)

or a pharmaceutically acceptable salt thereof.

**3.** A compound of Claim 2 which is:
2(S)-hydroxy-3(R)-(2-carboxyethylsulfinyl)-3-[2-(8-phenyloctyl)phenyl]propionic acid;
2(S)-hydroxy-3(R)-(2-carboxyethylsulfonyl-3-[2-(8-phenyloctyl)phenyl]propionic acid; or
the diarginine salt, disodium salt, dimagnesium salt, dicalcium salt, or diammonium salt thereof.

**4.** A pharmaceutical composition for inhibiting the effects of leukotriene comprising a pharmaceutical carrier or diluent and a nontoxic amount sufficient to produce said inhibition of a compound of Claim 1, formula (I).

**5.** A pharmaceutical composition for inhibiting antigen-induced respiratory anaphylaxis comprising a pharmaceutical carrier or diluent and nontoxic amounts sufficient to produce said inhibition of a compound of Claim 1, formula (I), and an histamine $H_1$-receptor antagonist.

**6.** A pharmaceutical composition according to Claim 5 in which the active ingredients are 2(S)-hydroxy-3-(R)-(2-carboxyethylsulfinyl)-3-[2-(8-phenyloctyl)phenyl]-propionic acid, 2(S)-hydroxy-3(R)-(2-carboxyethylsulfonyl)-3-[2-(8-phenyloctyl)phenyl]-propionic acid, or a pharmaceutically acceptable salt thereof, and 2-[4-(5-bromo-3-methylpyrid-2-yl)butylamino]-5-[(6-methylpyrid-3-yl)methyl]-4-pyrimidone.

**7.** A compound of Claim 1, formula (I) for use as a leukotriene antagonist, or for use in the treatment of diseases in which leukotrienes are a factor.

**8.** The use of a compound of Claim 1, formula (I), in the manufacture of a medicament having leukotriene antagonist activity.

**9.** A compound represented by the following general structural formula (I):

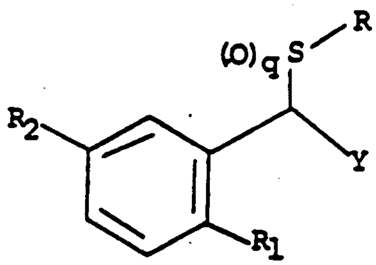

(I)

wherein q is 1 or 2;

$R_1$ is $C_8$ to $C_{13}$ alkyl, $C_7$ to $C_{12}$ alkoxy, $C_{10}$ to $C_{12}$ 1-alkynyl, 10-undecynyloxy, 11-dodecynyl, phenyl-$C_4$ to $C_{10}$ alkyl, phenyl-$C_3$ to $C_9$ alkoxy with the phenyl optionally mono substituted with bromo, chloro, trifluoromethyl, $C_1$ to $C_4$ alkoxy, methylthio or trifluoromethylthio; thienyl-$C_4$ to $C_{10}$ alkyl furyl-$C_4$ to $C_{10}$ alkyl, trifluoromethyl-$C_7$ to $C_{12}$ alkyl or cycloheyl-$C_4$ to $C_{10}$ alkyl;

$R_2$ is hydrogen, bromo, chloro, methyl, trifluoromethyl, hydroxy, $C_1$ to $C_4$ alkoxy or nitro; or $R_1$ is hydrogen and $R_2$ is $C_8$ to $C_{13}$ alkyl, $C_7$ to $C_{12}$ alkoxy, $C_{10}$ to $C_{12}$ 1-alkynyl, 10-undecynyloxy, 11-dodecynyl, phenyl-$C_4$ to $C_{10}$ alkyl, phenyl-$C_3$ to $C_9$ alkoxy with the phenyl optionally mono substituted with bromo, chloro, trifluoromethyl, $C_1$ to $C_4$ alkoxy, methylthio or trifluoromethylthio; furyl-$C_4$ to $C_{10}$ alkyl, trifluoromethyl-$C_7$ to $C_{12}$ alkyl or cyclohexyl-$C_4$ to $C_{10}$ alkyl;

Y is $COR_3$,

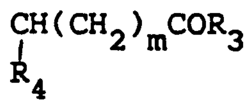

or $(CH_2)_{0-1}$-C-tetrazolyl;

$R_3$ is $C_1$ to $C_6$ alkoxy or hydroxy;

$R_4$ is hydrogen, methyl, $C_1$ to $C_4$ alkoxy, fluoro, or hydroxy;

m is 0, 1 or 2;

R is

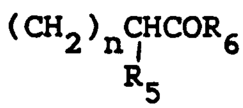

$CH(CO_2H)CH_2CO_2H$, $CH_2CH_2Z$ or

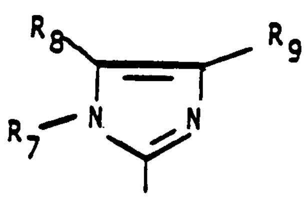

;

n is 0 to 6;

$R_5$ is hydrogen, amino, or $NHCOCH_2CH_2CH(NH_2)CO_2H$;

$R_6$ is hydroxy, or $C_1$ to $C_6$ alkoxy;

Z is $SO_3H$, $SO_2NH_2$ or CN;

$R_7$ is hydrogen, $C_1$ to $C_4$ alkyl, or $C_3$ to $C_4$ alkenyl;

$R_8$ is hydrogen, $C_1$ to $C_4$ alkyl, carboxyl or carboxamido, or $(CH_2)_pCO_2R_{12}$, wherein p is 1 or 2, $R_{12}$ is $C_1$ to $C_6$ alkyl or hydrogen, where $R_7$ and $R_9$ are hydrogen or $C_1$ to $C_4$ alkyl; and

$R_9$ is hydrogen, $C_1$ to $C_4$ alkyl, or $CH_2CO_2R_{13}$ wherein $R_{13}$ is $C_1$ to $C_6$ alkyl or hydrogen, with the proviso that (1) when n is 0, $R_5$ is hydrogen, (2) $R_7$, $R_8$ and $R_9$ are not all hydrogen; (3) $R_3$ and $R_6$ are not both hydroxy, or (4) $R_3$ is not hydroxy if both $R_{12}$ and $R_{13}$ are hydrogen, or a pharmaceutically acceptable salt thereof.

**10.** A process for the preparation of compounds of Claim 1, formula (I), which comprises reacting an appropriately protected substituted thiol, RSH, wherein R is as defined in Claim 1, with:

(a) a compound of the general formula:

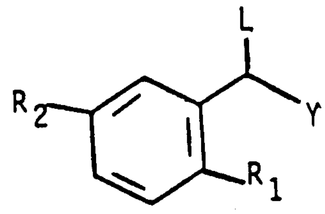

wherein $R_1$ and $R_2$ are as defined in Claim 1, L is a leaving group selected from chloro, bromo or hydroxy; Y is $CO_2R_{10}$ or Y is $CH(R_{12})CO_2R_{10}$ provided that $R_2$ is not $CF_3$, wherein $R_{10}$ is an ester protective group and $R_{12}$ is hydrogen, methyl, methoxy or fluoro, to form compounds wherein Y is $CO_2H$ or $CH(R_{12})CO_2H$;

(b) a compound of the general formula:

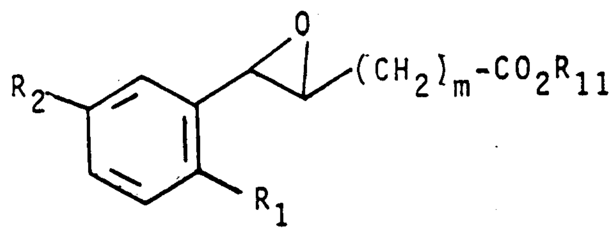

wherein $R_1$, $R_2$ and m are as defined in Claim 1, and $R_{11}$ is lower alkyl, to form compounds wherein Y is $CH(OH)\ (CH_2)_mCO_2H$;

(c) a compound of the general formula:

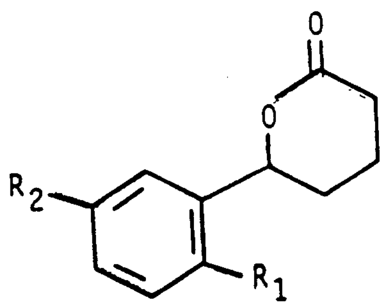

wherein $R_1$ and $R_2$ are as defined in claim 1 to form compounds wherein Y is $(CH_2)_3CO_2H$; and

(d) a compound of the general formula:

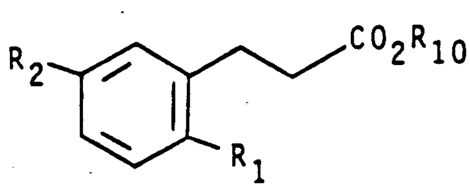

wherein $R_1$ and $R_2$ are as defined in claim 1 and $R_{10}$ is an ester protective group, to form compounds wherein Y is $CH_2CO_2H$;

followed by deprotection of any group, optionally resolving any diastereomeric mixture of compounds, oxidizing the sulfide group, and optionally forming a pharmaceutically acceptable salt.

**Claims for the following Contracting States : ES, GR**

**1.** A process for the preparation of compounds of the following structural formula (I):

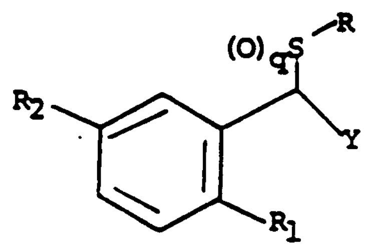

(I)

wherein

q is 1 or 2;

$R_1$ is $C_8$ to $C_{13}$ alkyl, $C_7$ to $C_{12}$ alkoxy, $C_{10}$ to $C_{12}$ 1-alkynyl, 10-undecynyloxy, 11-dodecynyl, phenyl-$C_4$ to $C_{10}$ alkyl, phenyl- $C_3$ to $C_9$ alkoxy with the phenyl optionally mono substituted with bromo, chloro, trifluoromethyl, $C_1$ to $C_4$ alkoxy, methylthio or trifluoromethylthio, furyl-$C_4$ to $C_{10}$ alkyl, trifluoromethyl-$C_7$ to $C_{12}$ alkyl or cyclohexyl-$C_4$ to $C_{10}$ alkyl;

$R_2$ is hydrogen, bromo, chloro, methyl, trifluoromethyl, hydroxy, $C_1$ to $C_4$ alkoxy or nitro; or $R_1$ is hydrogen and $R_2$ is $C_8$ to $C_{13}$ alkyl, $C_7$ to $C_{12}$ alkoxy, $C_{10}$ to $C_{12}$-1-alkynyl, 10-undecynyloxy, 11-dodecynyl, phenyl-$C_4$ to $C_{10}$-alkyl, phenyl-$C_3$ to $C_9$ alkoxy with the phenyl optionally mono substituted with bromo, chloro, trifluoromethyl, $C_1$ to $C_4$ alkoxy, methylthio or trifluoromethylthio, furyl-$C_4$ to $C_{10}$-alkyl, trifluoromethyl-$C_7$ to $C_{12}$ alkyl or cyclohexyl-$C_4$ to $C_{10}$ alkyl;

Y is $COR_3$,

$$\underset{R_4}{CH}(CH_2)_mCOR_3$$

or $(CH_2)_{0-1}$-C-tetrazolyl;

$R_3$ is hydroxy or amino;

$R_4$ is hydrogen, methyl, $C_1$ to $C_4$ alkoxy, fluoro or hydroxy;

m is 0, 1, or 2;

R is

$$(CH_2)_n\underset{R_5}{CH}COR_6,$$

$CH(CO_2H)CH_2CO_2H$, $CH_2CH_2Z$ or

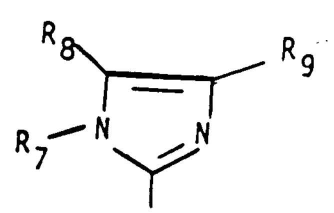

;

n is 0 to 6;

$R_5$ is hydrogen, amino, or $NHCOCH_2CH_2CH(NH_2)CO_2H$;

$R_6$ is hydroxy, amino or $NHCH_2CO_2H$;

Z is $SO_3H$, $SO_2NH_2$ or CN;

$R_7$ is hydrogen, $C_1$ to $C_4$ alkyl or $C_3$ to $C_4$ alkenyl;

$R_8$ is hydrogen, $C_1$ to $C_4$ alkyl, carboxyl or carboxamido, or $(CH_2)_pCO_2H$, wherein p is 1 or 2, when $R_7$ and $R_9$ are hydrogen or $C_1$ to $C_4$ alkyl; and

$R_9$ is hydrogen, $C_1$ to $C_4$ alkyl or $CH_2CO_2H$, with the proviso that when n is 0, $R_5$ is hydrogen and further that $R_7$, $R_8$ and $R_9$ are not all hydrogen; or a pharmaceutically acceptable salt thereof; which comprises reacting an appropriately protected substituted thiol, RSH, wherein R is as defined above with:

(a) a compound of the general formula:

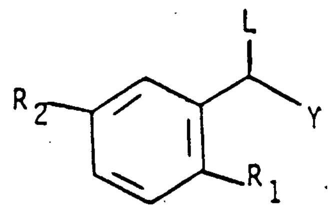

wherein $R_1$ and $R_2$ are as defined in Claim 1, L is a leaving group selected from chloro, bromo or hydroxy; Y is $CO_2R_{10}$ or Y is $CH(R_{12})CO_2R_{10}$ provided that $R_2$ is not $CF_3$, wherein $R_{10}$ is an ester protective group and $R_{12}$ is hydrogen, methyl, methoxy or fluoro, to form compounds wherein Y is $CO_2H$ or $CH(R_{12})CO_2H$;

(b) a compound of the general formula:

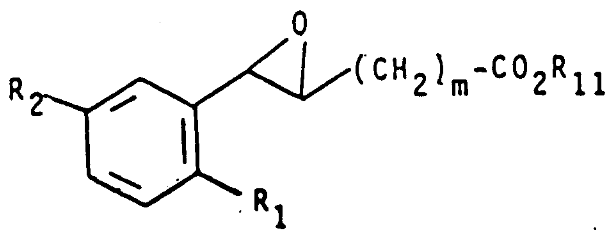

wherein $R_1$, $R_2$ and m are as defined in Claim 1, and $R_{11}$ is lower alkyl, to form compounds wherein Y is $CH(OH)(CH_2)_mCO_2H$;

(c) a compound of the general formula:

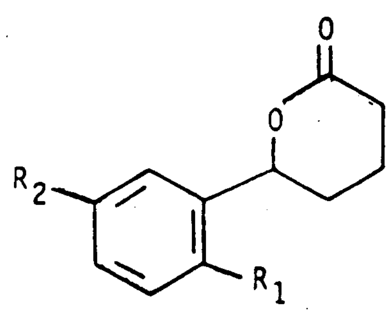

wherein $R_1$ and $R_2$ are as defined in claim 1 to form compounds wherein Y is $(CH_2)_3CO_2H$; and

(d) a compound of the general formula:

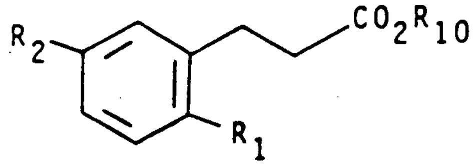

wherein $R_1$ and $R_2$ are as defined in claim 1 and $R_{10}$ is an ester protective group, to form

compounds wherein Y is $CH_2CO_2H$;

followed by deprotection of any group, optionally resolving any diastereomeric mixture of compounds oxidizing the sulfide group, and optionally forming a pharmaceutically acceptable salt.

**2.** A process for the preparation of 3-(2-carboxyethylsulfinyl)-3-[2-(8-phenyloctyl)phenyl]-2-hydroxypropionic acid which comprises reacting methyl 3-[2-(8-phenyloctyl)phenyl]-2,3-epoxypropionate with methyl 3-mercaptopropionate, followed by deprotection of the ester groups, and oxidation of the sulfur moiety.

**3.** A process for the preparation of 2(S)-hydroxy-3(R)-(2-carboxyethylsulfinyl)-3-[2-(8-phenyloctyl)phenyl]-propionic acid which comprises resolving the erythro mixture of diastereomers prior to oxidation of the sulfur moiety.

**4.** A process for the preparation of 3-(2-carboxyethylsulfonyl)-3-[2-(8-phenyloctyl)phenyl]-2-hydroxypropionic acid which comprises reacting methyl 3-[2-(8-phenyloctyl)phenyl]-2,3-epoxypropionate with methyl 3-mercaptopropionate, followed by deprotection of the ester groups, and oxidation of the sulfur moiety.

**5.** A process for the preparation of 2(S)-hydroxy-3(R)-(2-carboxyethylsulfonyl)-3-[2-(8-phenyloctyl)phenyl]-propionic acid which comprises resolving the erythro mixture of diastereomers prior to oxidation of the sulfur moiety.

**Revendications**

**Revendications pour les Etats contractants suivants : AT, BE, CH, DE, FR, GB, IT, LI, LU, NL, SE**

**1.** Composé représenté par la formule développée (I) suivante :

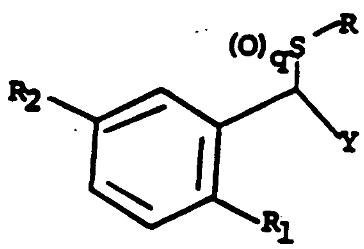

(I)

dans laquelle

q est égal à 1 ou 2;

$R_1$ est un groupe alkyle en $C_8$ à $C_{13}$, alcoxy en $C_7$ à $C_{12}$, 1-alcynyle en $C_{10}$ à $C_{12}$, 10-undécynyloxy, 11-dodécynyle, phényl-alkyle en $C_4$ à $C_{10}$, phényl-alcoxy en $C_3$ à $C_9$, le groupe phényle étant éventuellement mono-substitué par un groupe bromo, chloro, trifluorométhyle, alcoxy en $C_1$ à $C_4$, méthylthio ou trifluorométhylthio, furyl-alkyle en $C_4$ à $C_{10}$, trifluorométhyl-alkyle en $C_7$ à $C_{12}$ ou cyclohexyl-alkyle en $C_4$ à $C_{10}$;

$R_2$ est un hydrogène, ou un groupe bromo, chloro, méthyle, trifluorométhyle, hydroxy, alcoxy en $C_1$ à $C_4$ ou nitro; ou bien $R_1$ est un hydrogène et $R_2$ est un groupe alkyle en $C_8$ à $C_{13}$, alcoxy en $C_7$ à $C_{12}$, 1-alcynyle en $C_{10}$ à $C_{12}$, 10-undécynyloxy, 11-dodécynyle, phényl-alkyle en $C_4$ à $C_{10}$, phényl-alcoxy en $C_3$ à $C_9$, le groupe phényle étant éventuellement mono-substitué par un groupe bromo, chloro, trifluorométhyle, alcoxy en $C_1$ à $C_4$, méthylthio ou trifluorométhylthio, furyl-alkyle en $C_4$ à $C_{10}$, trifluorométhyl-alkyle en $C_7$ à $C_{12}$ ou cyclohexyl-alkyle en $C_4$ à $C_{10}$;

Y est $COR_3$,

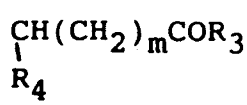

ou $(CH_2)_{0-1}$-C-tétrazolyle;

$R_3$ est un groupe hydroxy, amino;

$R_4$ est un hydrogène, ou un groupe méthyle, alcoxy en $C_1$ à $C_4$, fluoro ou hydroxy;

m est égal à 0, 1 ou 2;
R est

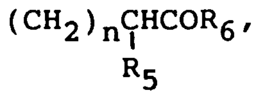

$CH(CO_2H)CH_2CO_2H$, $CH_2CH_2Z$ ou

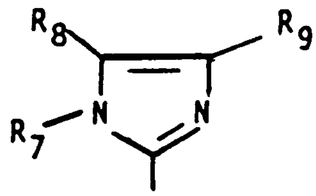

;

n a une valeur de 0 à 6;
$R_5$ est un hydrogène, ou un groupe amino ou $NHCOCH_2CH_2CH(NH_2)CO_2H$;
$R_6$ est un groupe hydroxy, amino ou $NHCH_2CO_2H$;
Z est $SO_3H$, $SO_2NH_2$ ou CN;
$R_7$ est un hydrogène, ou un groupe alkyle en $C_1$ à $C_4$ ou alcényle en $C_3$ à $C_4$;
$R_8$ est un hydrogène, ou un groupe alkyle en $C_1$ à $C_4$, carboxyle ou carboxamido, ou $(CH_2)_pCO_2H$, dans lequel p est égal à 1 ou 2, lorsque $R_7$ et $R_9$ sont des atomes d'hydrogène ou des groupes alkyle en $C_1$ à $C_4$; et
$R_9$ est un hydrogène, ou un groupe alkyle en $C_1$ à $C_4$ ou $CH_2CO_2H$, à condition que, lorsque n est nul, $R_5$ soit un hydrogène et qu'en outre $R_7$, $R_8$ et $R_9$ ne soient pas tous des atomes d'hydrogène; ou un de ses sels pharmaceutiquement acceptables.

**2.** Composé selon la revendication 1, représenté par la formule développée (II) suivante :

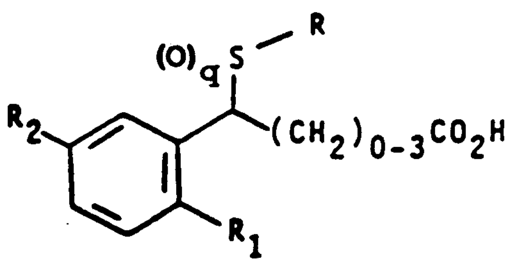

(II)

dans laquelle R est $(CH_2)_{1-3}CO_2H$ ou

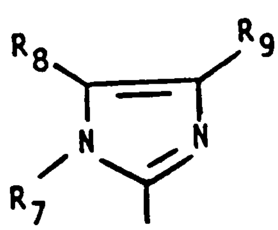

;

ou la formule développée (III) suivante :

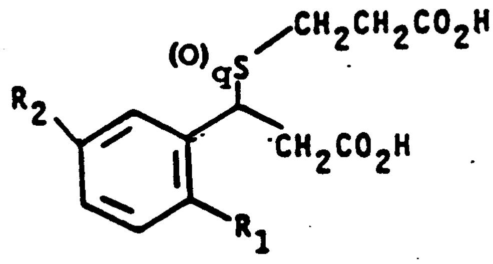

(III)

dans laquelle $R_1$ est un radical phényl-alkyle en $C_4$ à $C_{10}$; ou la formule développée (IV) suivante :

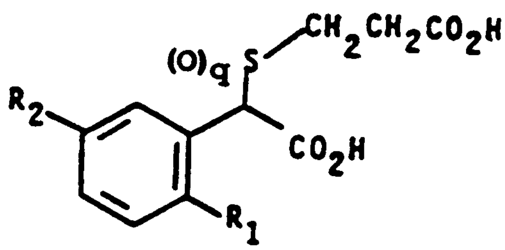

(IV)

dans laquelle $R_1$ est un radical phényl-alkyle en $C_4$ à $C_{10}$; ou la formule développée (V) suivante :

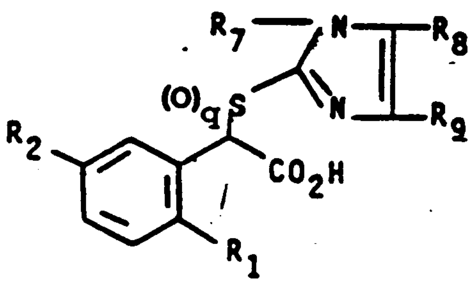

(V)

ou la formule développée (VI) suivante :

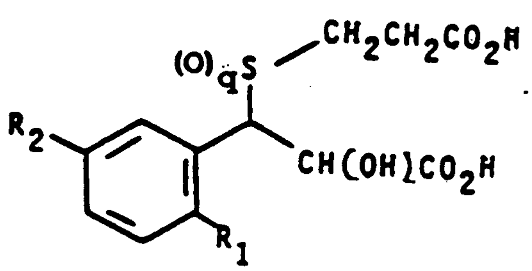

(VI)

dans laquelle $R_1$ est un radical phényl-alkyle en $C_4$ à $C_{10}$; ou la formule développée (VII) suivante :

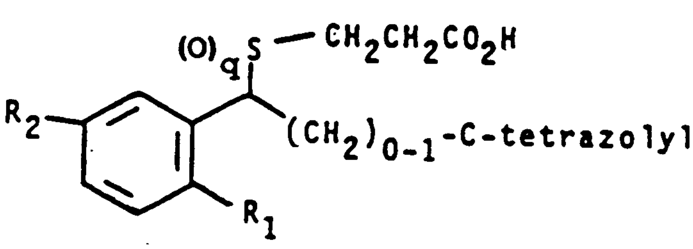

(VII)

ou un de ses sels pharmaceutiquement acceptables.

3. Composé selon la revendication 2, qui est
   l'acide 2(S)-hydroxy-3(R)-(2-carboxyéthylsulfinyl)-3-[2-(8-phényloctyl)phényl]propionique;
   l'acide 2(S)-hydroxy-3(R)-(2-carboxyéthylsulfonyl)-3-[2-(8-phényloctyl)phényl]propionique; ou
   leurs sels de diarginine, leurs sels disodiques, leurs sels dimagnésiques, leurs sels dicalciques ou leurs sels de diammonium.

4. Composition pharmaceutique servant à inhiber les effets du leucotriène, comprenant un véhicule ou diluant pharmaceutique et une quantité non toxique, suffisante pour produire ladite inhibition, d'un composé de formule (I) de la revendication 1.

5. Composition pharmaceutique servant à inhiber l'anaphylaxie respiratoire déclenchée par un antigène, comprenant un véhicule ou diluant pharmaceutique et des quantités non toxiques, suffisantes pour produire ladite inhibition, d'un composé de formule (I) de la revendication 1 et d'un antagoniste des récepteurs $H_1$ à l'histamine.

6. Composition pharmaceutique selon la revendication 5, dans laquelle les ingrédients actifs sont l'acide 2(S)-hydroxy-3(R)-(2-carboxyéthylsulfinyl)-3-[2-(8-phényloctyl)phényl]propionique, l'acide 2(S)-hydroxy-3(R)-(2-carboxyéthylsulfonyl)-3-[2-(8-phényloctyl)phényl]propionique, ou un de leurs sels pharmaceutiquement acceptables, et la 2-[4-(5-bromo-3-méthyl-pyri-2-yl)butylamino]-5-[(6-méthylpyrid-3-yl]méthyl]-4-pyrimidone.

7. Composé de formule (I) selon la revendication 1, utilisable comme antagoniste des leucotriènes ou utilisable dans le traitement de maladies dans lesquelles les leucotriènes sont un facteur.

8. Utilisation d'un composé de formule (I) selon la revendication 1 dans la fabrication d'un médicament ayant une activité antagoniste des leucotriènes.

9. Composé représenté par la formule générale développée (I) suivante :

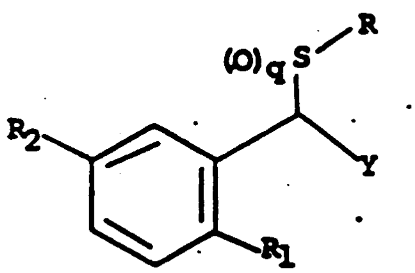

(I)

dans laquelle
   q est égal à 1 ou 2;
   $R_1$ est un groupe alkyle en $C_8$ à $C_{13}$, alcoxy en $C_7$ à $C_{12}$, 1-alcynyle en $C_{10}$ à $C_{12}$, 10-undécynyloxy, 11-dodécynyle, phényl-alkyle en $C_4$ à $C_{10}$, phényl-alcoxy en $C_3$ à $C_9$, le groupe phényle étant éventuellement mono-substitué par un groupe bromo, chloro, trifluorométhyle, alcoxy en $C_1$ à $C_4$, méthylthio ou trifluorométhylthio; thiényl-alkyle en $C_4$ à $C_{10}$, furyl-alkyle en $C_4$ à $C_{10}$, trifluorométhyl-alkyle en $C_7$ à $C_{12}$ ou cyclohexyl-alkyle en $C_4$ à $C_{10}$;
   $R_2$ est un hydrogène, ou un groupe bromo, chloro, méthyle, trifluorométhyle, hydroxy, alcoxy en $C_1$ à $C_4$ ou nitro; ou bien $R_1$ est un hydrogène et $R_2$ est un groupe alkyle en $C_8$ à $C_{13}$, alcoxy en $C_7$ à $C_{12}$, 1-alcynyle en $C_{10}$ à $C_{12}$, 10-undécynyloxy, 11-dodécynyle, phényl-alkyle en $C_4$ à $C_{10}$, phényl-alcoxy en $C_3$ à $C_9$, le groupe phényle étant éventuellement mono-substitué par un groupe bromo, chloro, trifluorométhyle, alcoxy en $C_1$ à $C_4$, méthylthio ou trifluorométhylthio; furyl-alkyle en $C_4$ à $C_{10}$, trifluorométhyl-alkyle en $C_7$ à $C_{12}$ ou cyclohexyl-alkyle en $C_4$ à $C_{10}$;
   Y est $COR_3$,

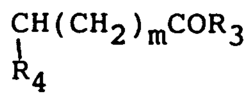

ou $(CH_2)_{0-1}$-C-tétrazolyle;

$R_3$ est un groupe alcoxy en $C_1$ à $C_6$ ou hydroxy;

$R_4$ est un hydrogène, ou un groupe méthyle, alcoxy en $C_1$ à $C_4$, fluoro ou hydroxy;

m est égal à 0, 1 ou 2;

R est

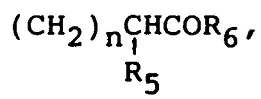

$CH(CO_2H)CH_2CO_2H$, $CH_2CH_2Z$ ou

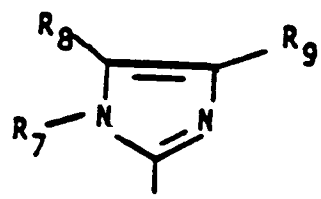 ;

n a une valeur de 0 à 6;

$R_5$ est un hydrogène, ou un groupe amino ou $NHCOCH_2CH_2CH(NH_2)CO_2H$;

$R_6$ est un groupe hydroxy, ou alcoxy en $C_1$ à $C_6$;

Z est $SO_3H$, $SO_2NH_2$ ou CN;

$R_7$ est un hydrogène, ou un groupe alkyle en $C_1$ à $C_4$ ou alcényle en $C_3$ à $C_4$;

$R_8$ est un hydrogène, ou un groupe alkyle en $C_1$ à $C_4$, carboxyle ou carboxamido, ou $(CH_2)_pCO_2R_{12}$, dans lequel p est égal à 1 ou 2, $R_{12}$ est un groupe alkyle en $C_1$ à $C_6$, ou un hydrogène, lorsque $R_7$ et $R_9$ sont des atomes d'hydrogène ou des groupes alkyle en $C_1$ à $C_4$; et

$R_9$ est un hydrogène, ou un groupe alkyle en $C_1$ à $C_4$ ou $CH_2CO_2R_{13}$, dans lequel $R_{13}$ est un groupe alkyle en $C_1$ à $C_6$ ou un hydrogène, à condition que, (1) lorsque n est nul, $R_5$ soit un hydrogène, (2) $R_7$, $R_8$ et $R_9$ ne soient pas tous des atomes d'hydrogène, (3) $R_3$ et $R_6$ ne soient pas tous deux des groupes hydroxy, ou (4) $R_3$ ne soit pas un groupe hydroxy si $R_{12}$ et $R_{13}$ sont tous deux des atomes d'hydrogène; ou un de ses sels pharmaceutiquement acceptables.

**10.** Procédé de préparation des composés de formule (I) selon la revendication 1, qui comprend la réaction d'un thiol substitué protégé de façon appropriée, RSH, où R est tel que défini dans la revendication 1, avec :

(a) un composé de formule générale :

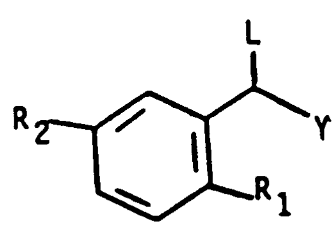

dans laquelle $R_1$ et $R_2$ sont tels que définis dans la revendication 1, L est un groupe partant choisi parmi un chloro, un bromo ou un hydroxy; Y est $CO_2R_{10}$ ou Y est $CH(R_{12})CO_2R_{10}$, à condition que $R_2$ ne soit pas $CF_3$, où $R_{10}$ est un groupe protecteur d'ester et $R_{12}$ est un hydrogène, ou un groupe méthyle, méthoxy ou fluoro, pour former des composés dans lesquels Y est $CO_2H$ ou $CH(R_{12})$-

$CO_2H$;

(b) un composé de formule générale :

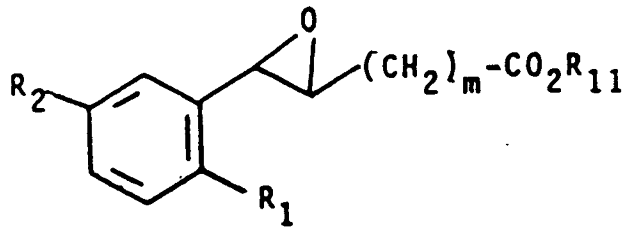

dans laquelle $R_1$, $R_2$ et m sont tels que définis dans la revendication 1 et $R_{11}$ est un groupe alkyle inférieur, pour former des composés dans lesquels Y est $CH(OH)(CH_2)_mCO_2H$;

(c) un composé de formule générale :

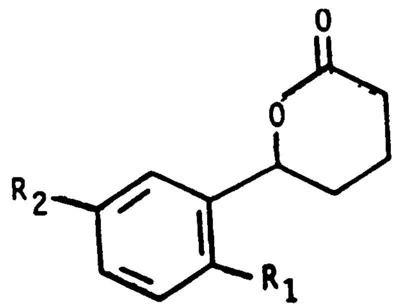

dans laquelle $R_1$ et $R_2$ sont tels que définis dans la revendication 1, pour former des composés dans lesquels Y est $(CH_2)_3CO_2H$; et

(d) un composé de formule générale :

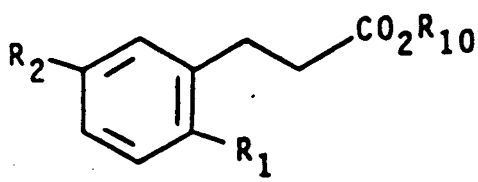

dans laquelle $R_1$ et $R_2$ sont tels que définis dans la revendication 1 et $R_{10}$ est un groupe protecteur d'ester, pour former des composés dans lesquels Y est $CH_2CO_2H$;

suivie de la déprotection éventuelle des groupes, éventuellement de la résolution du mélange diastéréoisomère de composés, de l'oxydation du groupe sulfure et, éventuellement, de la formation d'un sel pharmaceutiquement acceptable.

**Revendications pour les Etats contractants suivants : ES, GR**

**1.** Procédé de préparation de composés représentés par la formule développée (I) suivante :

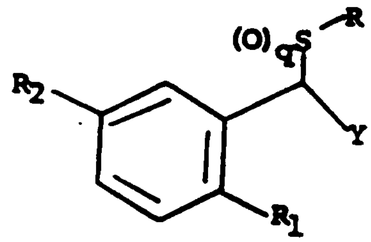

(I)

dans laquelle

q est égal à 1 ou 2;

$R_1$ est un groupe alkyle en $C_8$ à $C_{13}$, alcoxy en $C_7$ à $C_{12}$, 1-alcynyle en $C_{10}$ à $C_{12}$, 10-undécynyloxy, 11-dodécynyle, phényl-alkyle en $C_4$ à $C_{10}$, phényl-alcoxy en $C_3$ à $C_9$, le groupe phényle étant éventuellement mono-substitué par un groupe bromo, chloro, trifluorométhyle, alcoxy en $C_1$ à $C_4$, méthylthio ou trifluorométhylthio, furyl-alkyle en $C_4$ à $C_{10}$, trifluorométhyl-alkyle en $C_7$ à $C_{12}$ ou cyclohexyl-alkyle en $C_4$ à $C_{10}$;

$R_2$ est un hydrogène, ou un groupe bromo, chloro, méthyle, trifluorométhyle, hydroxy, alcoxy en $C_1$ à $C_4$ ou nitro; ou bien $R_1$ est un hydrogène et $R_2$ est un groupe alkyle en $C_8$ à $C_{13}$, alcoxy en $C_7$ à $C_{12}$, 1-alcynyle en $C_{10}$ à $C_{12}$, 10-undécynyloxy, 11-dodécynyle, phényl-alkyle en $C_4$ à $C_{10}$, phényl-alcoxy en $C_3$ à $C_9$, le groupe phényle étant éventuellement mono-substitué par un groupe bromo, chloro, trifluorométhyle, alcoxy en $C_1$ à $C_4$, méthylthio ou trifluorométhylthio, furyl-alkyle en $C_4$ à $C_{10}$, trifluorométhyl-alkyle en $C_7$ à $C_{12}$ ou cyclohexyl-alkyle en $C_4$ à $C_{10}$;

Y est $COR_3$,

$$\underset{R_4}{CH}(CH_2)_mCOR_3$$

ou $(CH_2)_{0-1}$-C-tétrazolyle;

$R_3$ est un groupe hydroxy ou amino;

$R_4$ est un hydrogène, ou un groupe méthyle, alcoxy en $C_1$ à $C_4$, fluoro ou hydroxy;

m est égal à 0, 1 ou 2;

R est

$$(CH_2)_n\underset{R_5}{CH}COR_6,$$

$CH(CO_2H)CH_2CO_2H$, $CH_2CH_2Z$ ou

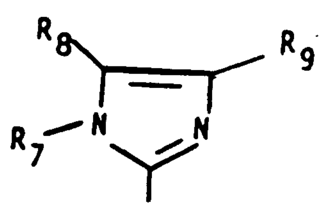

;

n a une valeur de 0 à 6;

$R_5$ est un hydrogène, ou un groupe amino ou $NHCOCH_2CH_2CH(NH_2)CO_2H$;

$R_6$ est un groupe hydroxy, amino ou $NHCH_2CO_2H$;

Z est $SO_3H$, $SO_2NH_2$ ou CN;

$R_7$ est un hydrogène, ou un groupe alkyle en $C_1$ à $C_4$ ou alcényle en $C_3$ à $C_4$;

$R_8$ est un hydrogène, ou un groupe alkyle en $C_1$ à $C_4$, carboxyle ou carboxamido, ou $(CH_2)_pCO_2H$ dans lequel p est égal à 1 ou 2, lorsque $R_7$ et $R_9$ sont des atomes d'hydrogène ou des groupes alkyle

en $C_1$ à $C_4$; et

$R_9$ est un hydrogène, ou un groupe alkyle en $C_1$ à $C_4$ ou $CH_2CO_2H$, à condition que, lorsque n est nul, $R_5$ soit un hydrogène et qu'en outre $R_7$, $R_8$ et $R_9$ ne soient pas tous des atomes d'hydrogène; ou d'un de leurs sels pharmaceutiquement acceptables;

qui comprend la réaction d'un thiol substitué protégé de façon appropriée, RSH, où R est tel que défini ci-dessus, avec :

(a) un composé de formule générale :

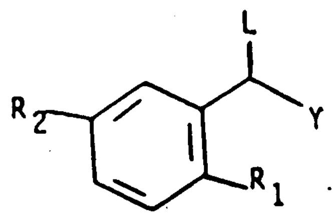

dans laquelle $R_1$ et $R_2$ sont tels que définis dans la revendication 1, L est un groupe partant choisi parmi un chloro, un bromo ou un hydroxy; Y est $CO_2R_{10}$ ou Y est $CH(R_{12})CO_2R_{10}$, à condition que $R_2$ ne soit pas $CF_3$, où $R_{10}$ est un groupe protecteur d'ester et $R_{12}$ est un hydrogène, ou un groupe méthyle, méthoxy ou fluoro, pour former des composés dans lesquels Y est $CO_2H$ ou $CH(R_{12})$-$CO_2H$;

(b) un composé de formule générale :

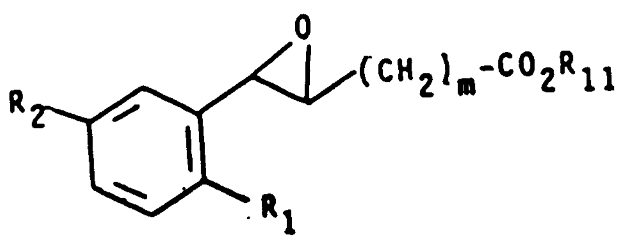

dans laquelle $R_1$, $R_2$ et m sont tels que définis dans la revendication 1 et $R_{11}$ est un groupe alkyle inférieur, pour former des composés dans lesquels Y est $CH(OH)(CH_2)_mCO_2H$;

(c) un composé de formule générale :

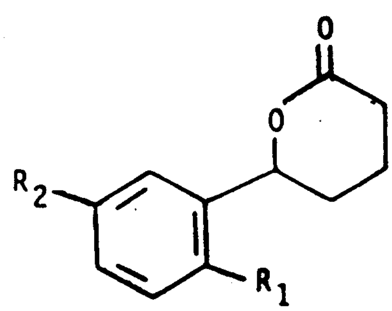

dans laquelle $R_1$ et $R_2$ sont tels que définis dans la revendication 1, pour former des composés dans lesquels Y est $(CH_2)_3CO_2H$; et

(d) un composé de formule générale :

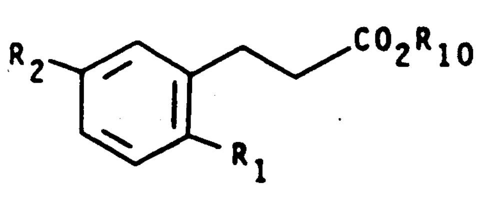

dans laquelle $R_1$ et $R_2$ sont tels que définis dans la revendication 1 et $R_{10}$ est un groupe protecteur d'ester, pour former des composés dans lesquels Y est $CH_2CO_2H$;

suivie de la déprotection éventuelle des groupes, éventuellement de la résolution du mélange diastéréoisomère de composés, de l'oxydation du groupe sulfure et, éventuellement, de la formation d'un sel pharmaceutiquement acceptable.

**2.** Procédé de préparation de l'acide 3-(2-carboxyéthylsulfinyl)-3-[2-(8-phényloctyl)phényl]-2-hydroxypropionique, qui comprend la réaction du 3-[2-(8-phényloctyl)phényl]-2,3-époxypropionate de méthyle avec le 3-mercaptopropionate de méthyle, suivie de la déprotection des groupes esters et de l'oxydation du groupement soufré.

**3.** Procédé de préparation de l'acide 2(S)-hydroxy-3(R)-(2-carboxyéthylsulfinyl)-3-[2-(8-phényloctyl)-phényl]propionique, qui comprend la résolution du mélange érythro de diastéréoisomères avant l'oxydation du groupement soufré.

**4.** Procédé de préparation de l'acide 3-(2-carboxyéthylsulfonyl)-3-[2-(8-phényloctyl)phényl]-2-hydroxypropionique, qui comprend la réaction du 3-[2-(8-phényloctyl)phényl]-2,3-époxypropionate de méthyle avec le 3-mercaptopropionate de méthyle, suivie de la déprotection des groupes esters et de l'oxydation du groupement soufré.

**5.** Procédé de préparation de l'acide 2(S)-hydroxy-3(R)-(2-carboxyéthylsulfonyl)-3-[2-(8-phényloctyl)-phényl]propionique, qui comprend la résolution du mélange érythro de diastéréoisomères avant l'oxydation du groupement soufré.

**Patentansprüche**
**Patentansprüche für folgende Vertragsstaaten : AT, BE, CH, DE, FR, GB, IT, LI, LU, NL, SE**

**1.** Verbindung der folgenden Strukturformel (I):

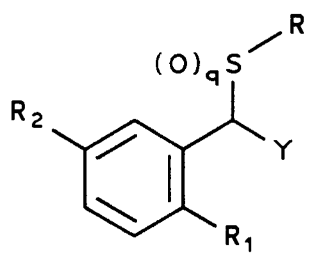

(I)

in der

q 1 oder 2 ist,

$R_1$ einen $C_8$- bis $C_{13}$-Alkyl-, $C_7$- bis $C_{12}$-Alkoxy-, $C_{10}$- bis $C_{12}$-1-Alkinylrest, eine 10-Undecinyloxy-, 11-Dodecinylgruppe, einen Phenyl-$C_4$- bis $C_{10}$-Alkyl-, Phenyl-$C_3$- bis $C_9$-Alkoxyrest, wobei die Phenylgruppe gegebenenfalls mit einem Brom-, Chloratom, einer Trifluormethylgruppe, einem $C_1$- bis $C_4$-Alkoxyrest, einer Methylthiooder einer Trifluormethylthiogruppe monosubstituiert ist, einen Furyl-$C_4$- bis $C_{10}$-Alkyl-, Trifluormethyl-$C_7$- bis $C_{12}$-Alkyl- oder einen Cyclohexyl-$C_4$- bis $C_{10}$-Alkylrest bedeutet;

$R_2$ ein Wasserstoff-, Brom-, Chloratom, eine Methyl-, Trifluormethyl-, Hydroxylgruppe, ein $C_1$- bis $C_4$-Alkoxyrest oder eine Nitrogruppe ist, oder $R_1$ ein Wasserstoffatom und $R_2$ einen $C_8$-bis $C_{13}$-Alkyl-, $C_7$- bis $C_{12}$-Alkoxy-, $C_{10}$- bis $C_{12}$-1-Alkinylrest, eine 10-Undecinyloxy-, 11-Dodecinylgruppe, einen Phenyl-$C_4$- bis $C_{10}$-Alkyl-, Phenyl-$C_3$- bis $C_9$-Alkoxyrest, wobei die Phenylgruppe gegebenenfalls mit einem Brom-, Chloratom, einer Trifluormethylgruppe, einem $C_1$- bis $C_4$-Alkoxyrest, einer Methylthio- oder einer Trifluormethylthiogruppe monosubstituiert ist, einen Furyl-$C_4$- bis $C_{10}$-Alkyl-,Trifluormethyl-$C_7$- bis $C_{12}$-Alkyl- oder einen Cyclohexyl-$C_4$- bis $C_{10}$-Alkylrest bedeutet;

Y ein Rest der Formel $COR_3$,

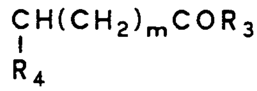

oder der Formel $(CH_2)_{0-1}$ C-Tetrazolyl ist;

$R_3$ eine Hydroxyl- oder eine Aminogruppe bedeutet;

$R_4$ ein Wasserstoffatom, eine Methylgruppe, ein $C_1$- bis $C_4$-Alkoxyrest, ein Fluoratom oder eine Hydroxylgruppe ist;

m 0, 1 oder 2 ist;

R einen Rest der Formel

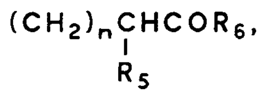

$CH(CO_2H)CH_2CO_2H$, $CH_2CH_2Z$ oder der Formel

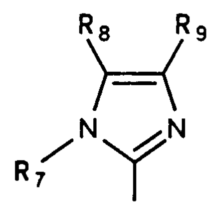

bedeutet;

n 0 bis 6 ist;

$R_5$ ein Wasserstoffatom, eine Aminogruppe oder einen Rest der Formel $NHCOCH_2CH_2CH(NH_2)$-$CO_2H$ bedeutet;

$R_6$ eine Hydroxyl-, Aminogruppe oder einen Rest der Formel $NHCH_2CO_2H$ bedeutet;

Z ein Rest der Formel $SO_3H$, $SO_2NH_2$ oder CN ist;

$R_7$ ein Wasserstoffatom, einen $C_1$- bis $C_4$-Alkyl- oder einen $C_3$- bis $C_4$-Alkenylrest bedeutet;

$R_8$ ein Wasserstoffatom, einen $C_1$- bis $C_4$-Alkylrest, eine Carboxyl- oder eine Carboxamidgruppe oder einen Rest der Formel $(CH_2)_pCO_2H$ bedeutet, in der p 1 oder 2 ist, falls $R_7$ und $R_9$ ein Wasserstoffatom oder einen $C_1$- bis $C_4$-Alkylrest bedeuten; und

$R_9$ ein Wasserstoffatom, ein $C_1$- bis $C_4$-Alkylrest oder ein Rest der Formel $CH_2CO_2H$ ist, mit der Maßgabe, daß, falls n 0 ist, $R_5$ ein Wasserstoffatom ist, und daß ferner $R_7$, $R_8$ und $R_9$ nicht alle ein Wasserstoffatom bedeuten; oder ein pharmazeutisch verträgliches Salz davon.

**2.** Verbindung nach Anspruch 1 der folgenden Strukturformel (II):

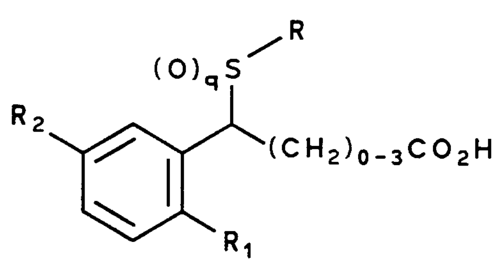

(II)

in der R einen Rest der Formel $(CH_2)_{1-3}CO_2H$ oder der Formel

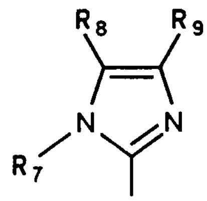

oder der folgenden Strukturformel (III):

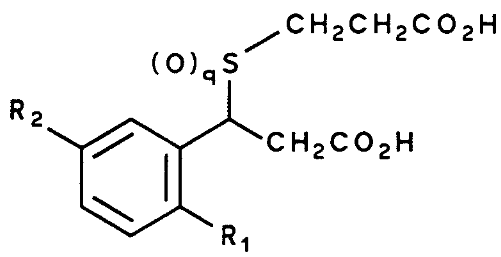

(III)

in der $R_1$ ein Phenyl-$C_4$- bis $C_{10}$-Alkylrest ist; oder der folgenden Strukturformel (IV):

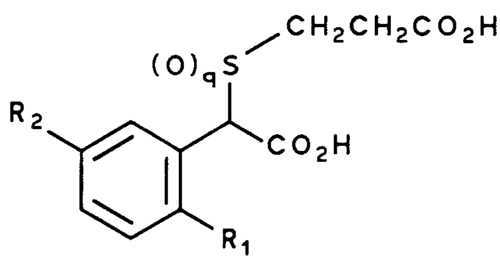

(IV)

in der $R_1$ ein Phenyl-$C_4$- bis $C_{10}$-Alkylrest ist; oder der folgenden Strukturformel (V):

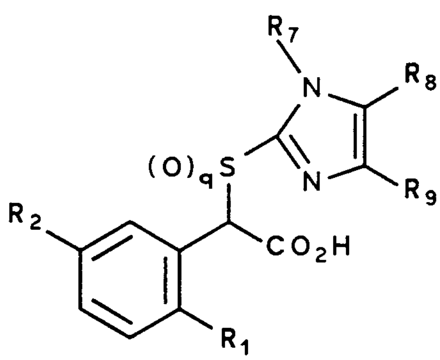

(V)

oder der folgenden Strukturformel (VI):

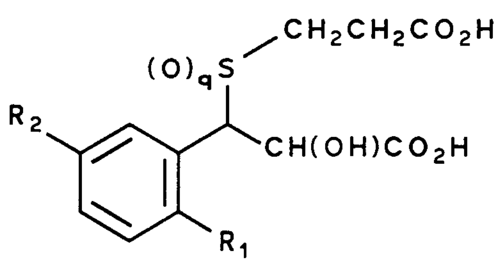

(VI)

in der $R_1$ ein Phenyl-$C_4$- bis $C_{10}$-Alkylrest ist; oder der folgenden Strukturformel (VII):

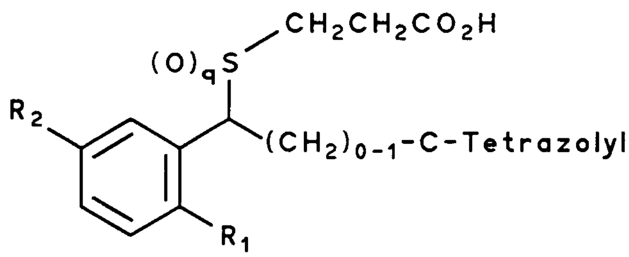

(VII)

bedeutet; oder ein pharmazeutisch verträgliches Salz davon.

**3.** Verbindung nach Anspruch 2, die 2(S)-Hydroxy-3(R)-(2-carboxyethylsulfinyl)-3-[2-(8-phenyloctyl)-phenyl]propionsäure, 2(S)-Hydroxy-3(R)-(2-carboxyethylsulfonyl)-3-[2-(8-phenyloctyl)phenyl]-propionsäure oder das Diarginin-, Dinatrium-, Dimagnesium-, Dicalcium- oder das Diammoniumsalz davon bedeutet.

**4.** Arzneimittel zur Inhibierung der Leukotrienwirkungen, umfassend einen pharmazeutischen Träger oder ein Verdünnungsmittel und eine nicht toxische, zur Erzeugung der Inhibierung ausreichende Menge einer Verbindung nach Anspruch 1, Formel (I).

**5.** Arzneimittel zur Inhibierung antigeninduzierter Atmungsanaphylaxie, umfassend einen pharmazeutischen Träger oder ein Verdünnungsmittel und nicht toxische, zur Erzeugung der Inhibierung ausreichende Mengen einer Verbindung nach Anspruch 1, Formel (I) und einen Histamin-$H_1$-Rezeptorantagonisten.

**6.** Arzneimittel gemäß Anspruch 5, wobei die Wirkstoffe 2(S)-Hydroxy-3(R)-(2-carboxyethylsulfinyl)-3-[2-(8-phenyloctyl)phenyl]propionsäure, 2(S)-Hydroxy-3(R)-(2-carboxyethylsulfonyl)-3-[2-(8-phenyloctyl)-phenyl]propionsäure oder ein pharmazeutisch verträgliches Salz davon und 2-[4-(5-Brom-3-methylpyrid-2-yl)butylamino]-5-[(6-methylpyrid-3-yl)methyl]-4-pyrimidon sind.

**7.** Verbindung nach Anspruch 1, Formel (I) zur Verwendung als Leukotrienantagonist oder zur Verwendung bei der Behandlung von Krankheiten, in denen Leukotriene ein Faktor sind.

**8.** Die Verwendung einer Verbindung nach Anspruch 1, Formel (I) bei der Herstellung eines Medikamentes mit Leukotrienantagonistenwirksamkeit.

**9.** Verbindung der folgenden allgemeinen Strukturformel (I):

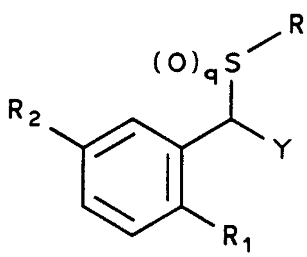

(I)

in der

q 1 oder 2 ist,

$R_1$ einen $C_8$- bis $C_{13}$-Alkyl-, $C_7$- bis $C_{12}$-Alkoxy-, $C_{10}$- bis $C_{12}$-1-Alkinylrest, eine 10-Undecinyloxy-, 11-Dodecinylgruppe, einen Phenyl-$C_4$- bis $C_{10}$-Alkyl-, Phenyl-$C_3$- bis $C_9$-Alkoxyrest, wobei die Phenylgruppe gegebenenfalls mit einem Brom-, Chloratom, einer Trifluormethylgruppe, einem $C_1$- bis $C_4$-Alkoxyrest, einer Methylthio- oder einer Trifluormethylthiogruppe monosubstituiert ist, einen Thienyl-$C_4$- bis $C_{10}$-Alkyl-, Furyl-$C_4$- bis $C_{10}$-Alkyl-, Trifluormethyl-$C_7$- bis $C_{12}$-Alkyl- oder einen Cyclohexyl-$C_4$- bis

$C_{10}$-Alkylrest bedeutet;

$R_2$ ein Wasserstoff-, Brom-, Chloratom, eine Methyl-, Trifluormethyl-, Hydroxylgruppe, ein $C_1$- bis $C_4$-Alkoxyrest oder eine Nitrogruppe ist, oder $R_1$ ein Wasserstoffatom und $R_2$ einen $C_8$- bis $C_{13}$-Alkyl-, $C_7$- bis $C_{12}$-Alkoxy-, $C_{10}$- bis $C_{12}$-1-Alkinylrest, eine 10-Undecinyloxy-, 11-Dodecinylgruppe, einen Phenyl-$C_4$- bis $C_{10}$-Alkyl-, Phenyl-$C_3$- bis $C_9$-Alkoxyrest, wobei die Phenylgruppe gegebenenfalls mit einem Brom-, Chloratom, einer Trifluormethylgruppe, einem $C_1$- bis $C_4$-Alkoxyrest, einer Methylthio- oder einer Trifluormethylthiogruppe monosubstituiert ist, einen Furyl-$C_4$- bis $C_{10}$-Alkyl-,Trifluormethyl-$C_7$- bis $C_{12}$-Alkyl- oder einen Cyclohexyl-$C_4$- bis $C_{10}$-Alkylrest bedeutet;

Y ein Rest der Formel $COR_3$,

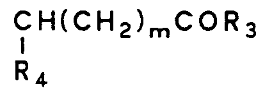

oder der Formel $(CH_2)_{0-1}$-C-Tetrazolyl ist;

$R_3$ einen $C_1$- bis $C_6$-Alkoxyrest oder eine Hydroxylgruppe bedeutet;

$R_4$ ein Wasserstoffatom, eine Methylgruppe, ein $C_1$- bis $C_4$-Alkoxyrest, ein Fluoratom oder eine Hydroxylgruppe ist;

m 0, 1 oder 2 ist;

R einen Rest der Formel

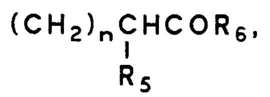

$CH(CO_2H)CH_2CO_2H$, $CH_2CH_2Z$ oder der Formel

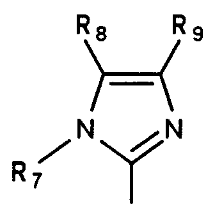

bedeutet;

n 0 bis 6 ist;

$R_5$ ein Wasserstoffatom, eine Aminogruppe oder einen Rest der Formel $NHCOCH_2CH_2CH(NH_2)$-$CO_2H$ bedeutet;

$R_6$ eine Hydroxylgruppe oder einen $C_1$- bis $C_6$-Alkoxyrest bedeutet;

Z ein Rest der Formel $SO_3H$, $SO_2NH_2$ oder CN ist;

$R_7$ ein Wasserstoffatom, einen $C_1$- bis $C_4$-Alkyl- oder einen $C_3$- bis $C_4$-Alkenylrest bedeutet;

$R_8$ ein Wasserstoffatom, einen $C_1$- bis $C_4$-Alkylrest, eine Carboxyl- oder eine Carboxamidgruppe oder einen Rest der Formel $(CH_2)_pCO_2R_{12}$ bedeutet, in der p 1 oder 2 ist, $R_{12}$ ein $C_1$- bis $C_6$-Alkylrest oder ein Wasserstoffatom ist, falls $R_7$ und $R_9$ ein Wasserstoffatom oder einen $C_1$- bis $C_4$-Alkylrest bedeuten; und

$R_9$ ein Wasserstoffatom, ein $C_1$- bis $C_4$-Alkylrest oder ein Rest der Formel $CH_2CO_2R_{13}$ ist, in der $R_{13}$ einen $C_1$- bis $C_6$-Alkylrest oder ein Wasserstoffatom bedeutet, mit der Maßgabe, daß (1) falls n 0 ist, $R_5$ ein Wasserstoffatom bedeutet, (2) $R_7$, $R_8$ und $R_9$ nicht alle ein Wasserstoffatom bedeuten, (3) $R_3$ und $R_6$ nicht beide eine Hydroxylgruppe sind oder (4) $R_3$ keine Hydroxylgruppe bedeutet, falls sowohl $R_{12}$ als auch $R_{13}$ ein Wasserstoffatom sind; oder ein pharmazeutisch verträgliches Salz davon.

**10.** Verfahren zur Herstellung der Verbindungen nach Anspruch 1, Formel (I), umfassend die Umsetzung eines geeignet geschützten, substituierten Thiols der Formel RSH, in der R wie in Anspruch 1 definiert ist, mit:

(a) einer Verbindung der allgemeinen Formel:

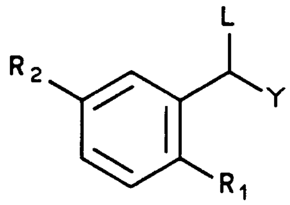

in der $R_1$ und $R_2$ wie in Anspruch 1 definiert sind, L eine austretende Gruppe, ausgewählt aus einem Chlor-, Bromatom oder einer Hydroxylgruppe, bedeutet, Y ein Rest der Formel $CO_2R_{10}$ oder $CH(R_{12})CO_2R_{10}$ ist, mit der Maßgabe, daß $R_2$ kein Rest der Formel $CF_3$ ist, in der $R_{10}$ eine Esterschutzgruppe bedeutet, und $R_{12}$ ein Wasserstoffatom, eine Methyl-, Methoxygruppe oder ein Fluoratom ist, wobei Verbindungen, in denen Y einen Rest der Formel $CO_2H$ oder $CH(R_{12})CO_2H$ bedeutet, gebildet werden;

(b) einer Verbindung der allgemeinen Formel:

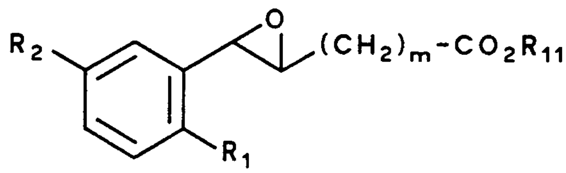

in der $R_1$, $R_2$ und m wie in Anspruch 1 definiert sind, und $R_{11}$ einen Niederalkylrest bedeutet, wobei Verbindungen, in denen Y ein Rest der Formel $CH(OH)(CH_2)_mCO_2H$ ist, gebildet werden;

(c) einer Verbindung der allgemeinen Formel:

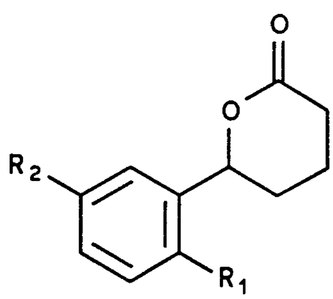

in der $R_1$ und $R_2$ wie in Anspruch 1 definiert sind, zur Bildung von Verbindungen, in denen Y einen Rest der Formel $(CH_2)_3CO_2H$ bedeutet; und

(d) einer Verbindung der allgemeinen Formel:

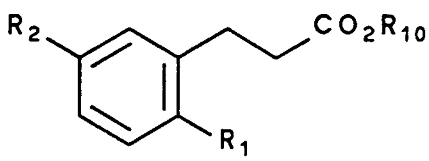

in der $R_1$ und $R_2$ wie in Anspruch 1 definiert sind, und $R_{10}$ eine Esterschutzgruppe ist, wobei Verbindungen, in denen Y einen Rest der Formel $CH_2CO_2H$ bedeutet, gebildet werden;

und anschließend Abspaltung aller Schutzgruppen, gegebenenfalls Trennung jedes diastereomeren Gemisches der Verbindungen, Oxidieren der Sulfidgruppe und gegebenenfalls Bildung eines pharmazeutisch verträglichen Salzes.

**Patentansprüche für folgende Vertragsstaaten : ES, GR**

**1.** Verfahren zur Herstellung von Verbindungen der folgenden Strukturformel (I):

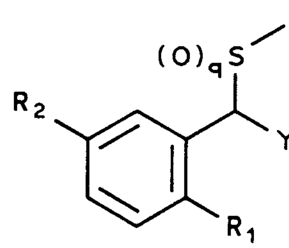

(I)

in der

q 1 oder 2 ist,

$R_1$ einen $C_8$- bis $C_{13}$-Alkyl-, $C_7$- bis $C_{12}$-Alkoxy-, $C_{10}$- bis $C_{12}$-1-Alkinylrest, eine 10-Undecinyloxy-, 11-Dodecinylgruppe, einen Phenyl-$C_4$- bis $C_{10}$-Alkyl-, Phenyl-$C_3$- bis $C_9$-Alkoxyrest, wobei die Phenylgruppe gegebenenfalls mit einem Brom-, Chloratom, einer Trifluormethylgruppe, einem $C_1$- bis $C_4$-Alkoxyrest, einer Methylthio- oder einer Trifluormethylthiogruppe monosubstituiert ist, einen Furyl-$C_4$- bis $C_{10}$-Alkyl-, Trifluormethyl-$C_7$- bis $C_{12}$-Alkyl- oder einen Cyclohexyl-$C_4$- bis $C_{10}$-Alkylrest bedeutet;

$R_2$ ein Wasserstoff-, Brom-, Chloratom, eine Methyl-, Trifluormethyl-, Hydroxylgruppe, ein $C_1$- bis $C_4$-Alkoxyrest oder eine Nitrogruppe ist, oder $R_1$ ein Wasserstoffatom und $R_2$ einen $C_8$- bis $C_{13}$-Alkyl-, $C_7$- bis $C_{12}$-Alkoxy-, $C_{10}$- bis $C_{12}$-1-Alkinylrest, eine 10-Undecinyloxy-, 11-Dodecinylgruppe, einen Phenyl-$C_4$- bis $C_{10}$-Alkyl-, Phenyl-$C_3$- bis $C_9$-Alkoxyrest, wobei die Phenylgruppe gegebenenfalls mit einem Brom-, Chloratom, einer Trifluormethylgruppe, einem $C_1$- bis $C_4$-Alkoxyrest, einer Methylthio- oder einer Trifluormethylthiogruppe monosubstituiert ist, einen Furyl-$C_4$- bis $C_{10}$-Alkyl-,Trifluormethyl-$C_7$- bis $C_{12}$-Alkyl- oder einen Cyclohexyl-$C_4$- bis $C_{10}$-Alkylrest bedeutet;

Y ein Rest der Formel $COR_3$,

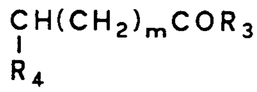

oder der Formel $(CH_2)_{0-1}$-C-Tetrazolyl ist;

$R_3$ eine Hydroxyl- oder eine Aminogruppe bedeutet;

$R_4$ ein Wasserstoffatom, eine Methylgruppe, ein $C_1$- bis $C_4$-Alkoxyrest, ein Fluoratom oder eine Hydroxylgruppe ist;

m 0, 1 oder 2 ist;

R einen Rest der Formel

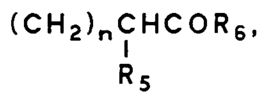

$CH(CO_2H)CH_2CO_2H$, $CH_2CH_2Z$ oder der Formel

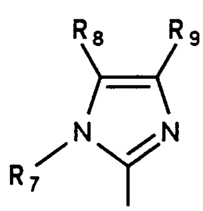

bedeutet;

n 0 bis, 6 ist;

$R_5$ ein Wasserstoffatom, eine Aminogruppe oder einen Rest der Formel $NHCOCH_2CH_2CH(NH_2)$-$CO_2H$ bedeutet;

$R_6$ eine Hydroxyl-, Aminogruppe oder einen Rest der Formel $NHCH_2CO_2H$ bedeutet;

Z ein Rest der Formel $SO_3H$, $SO_2NH_2$ oder CN ist;

$R_7$ ein Wasserstoffatom, einen $C_1$- bis $C_4$-Alkyl- oder einen $C_3$- bis $C_4$-Alkenylrest bedeutet;

$R_8$ ein Wasserstoffatom, einen $C_1$- bis $C_4$-Alkylrest, eine Carboxyl- oder eine Carboxamidgruppe oder einen Rest der Formel $(CH_2)_pCO_2H$ bedeutet, in der p 1 oder 2 ist, falls $R_7$ und $R_9$ ein Wasserstoffatom oder einen $C_1$- bis $C_4$-Alkylrest bedeuten; und

$R_9$ ein Wasserstoffatom, ein $C_1$- bis $C_4$-Alkylrest oder ein Rest der Formel $CH_2CO_2H$ ist, mit der Maßgabe, daß, falls n 0 ist, $R_5$ ein Wasserstoffatom ist, und daß ferner $R_7$, $R_8$ und $R_9$ nicht alle ein Wasserstoffatom bedeuten; oder eines pharmazeutisch verträglichen Salzes davon, umfassend die Umsetzung eines geeignet geschützten, substituierten Thiols der Formel RSH, in der R wie vorstehend definiert ist, mit:

(a) einer Verbindung der allgemeinen Formel:

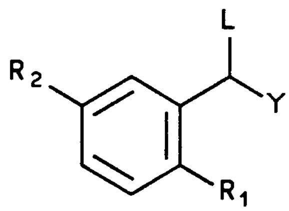

in der $R_1$ und $R_2$ wie in Anspruch 1 definiert sind, L eine austretende Gruppe, ausgewählt aus einem Chlor-, Bromatom oder einer Hydroxylgruppe, bedeutet, Y ein Rest der Formel $CO_2R_{10}$ oder $CH(R_{12})CO_2R_{10}$ ist, mit der Maßgabe, daß $R_2$ kein Rest der Formel $CF_3$ ist, in der $R_{10}$ eine Esterschutzgruppe bedeutet, und $R_{12}$ ein Wasserstoffatom, eine Methyl-, Methoxygruppe oder ein Fluoratom ist, wobei Verbindungen, in denen Y einen Rest der Formel $CO_2H$ oder $CH(R_{12})CO_2H$ bedeutet, gebildet werden;

(b) einer Verbindung der allgemeinen Formel:

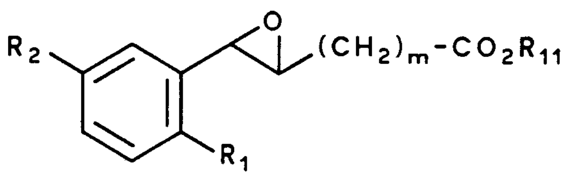

in der $R_1$, $R_2$ und m wie in Anspruch 1 definiert sind, und $R_{11}$ einen Niederalkylrest bedeutet, wobei Verbindungen, in denen Y ein Rest der Formel $CH(OH)(CH_2)_mCO_2H$ ist, gebildet werden;

(c) einer Verbindung der allgemeinen Formel:

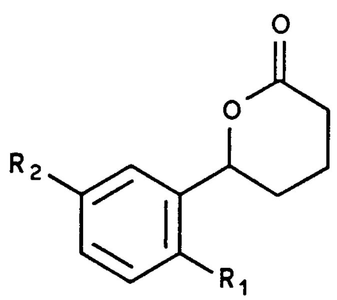

in der $R_1$ und $R_2$ wie in Anspruch 1 definiert sind, wobei Verbindungen, in denen Y einen Rest der Formel $(CH_2)_3CO_2H$ bedeutet, gebildet werden; und

(d) einer Verbindung der allgemeinen Formel:

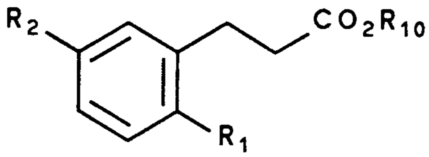

in der $R_1$ und $R_2$ wie in Anspruch 1 definiert sind, und $R_{10}$ eine Esterschutzgruppe ist, wobei Verbindungen, in denen Y einen Rest der Formel $CH_2CO_2H$ bedeutet, gebildet werden;
und anschließend Abspaltung aller Schutzgruppen, gegebenenfalls Trennung jedes diastereomeren Gemisches der Verbindungen, Oxidieren der Sulfidgruppe und gegebenenfalls Bildung eines pharmazeutisch verträglichen Salzes.

**2.** Verfahren zur Herstellung von 3-(2-Carboxyethylsulfinyl)-3-[2-(8-phenyloctyl)phenyl]-2-hydroxypropionsäure, umfassend die Umsetzung von Methyl-3-[2-(8-phenyloctyl)phenyl]-2,3-epoxypropionatmit Methyl-3-mercaptopropionat und anschließend Abspaltung der Esterschutzgruppen und die Oxidation der Schwefeleinheit.

**3.** Verfahren zur Herstellung von 2(S)-Hydroxy-3(R)-(2-carboxyethylsulfinyl)-3-[2-(8-phenyloctyl)phenyl]-propionsäure, umfassend die Trennung des erythro-Gemisches der Diastereomere vor der Oxidation der Schwefeleinheit.

**4.** Verfahren zur Herstellung von 3-(2-Carboxyethylsulfonyl)-3-[2-(8-phenyloctyl)phenyl]-2-hydroxypropionsäure, umfassend die Umsetzung von Methyl-3-[2-(8-phenyloctyl)phenyl]-2,3-epoxypropionatmit Methyl-3-mercaptopropionat und anschließend Abspaltung der Esterschutzgruppen und die Oxidation der Schwefeleinheit.

**5.** Verfahren zur Herstellung von 2(S)-Hydroxy-3(R)-(2-carboxyethylsulfonyl)-3-[2-(8-phenyloctyl)phenyl]-propionsäure, umfassend die Trennung des erythro-Gemisches der Diastereomere vor der Oxidation der Schwefeleinheit.